# EUROPÄISCHE PATENTSCHRIFT

(11) **EP 2 013 325 B1**
(45) Veröffentlichungstag und Bekanntmachung des Hinweises auf die Patenterteilung: **13.06.2012**
(21) Anmeldenummer: 07727725.9
(22) Anmeldetag: 03.04.2007
(51) Int. Cl.: C11D 3/39, C11D 17/00, D21C 9/16, A61K 8/38, A61K 8/11, A61Q 5/08

(54) **MIT POLYMEREN SCHICHTFÖRMIG UMHÜLLTE BLEICHSYSTEME**
BLEACH SYSTEMS ENVELOPED WITH POLYMERIC LAYERS
SYSTEME DE BLANCHIMENT ENROBE D'UNE COUCHE POLYMERE

(30) Priorität: 04.04.2006 EP 06112200
(43) Veröffentlichungstag der Anmeldung: 14.01.2009
(73) Patentinhaber: BASF SE, 67056 Ludwigshafen (DE)
(72) Erfinder: DIETSCHE, Frank, 69198 Schriesheim (DE); HÄBERLE, Karl, 67346 Speyer (DE); RAHN, Ralf-Thomas, 68167 Mannheim (DE); HÄRING, Dietmar, 68535 Neu-Edingen (DE)
(86) Internationale Anmeldenummer: PCT/EP2007/053254
(87) Internationale Veröffentlichungsnummer: WO 2007/115979

(56) Entgegenhaltungen:
- EP-A- 1 264 812
- EP-A1- 1 188 820
- WO-A-02/09862

## Beschreibung

Die vorliegende Erfindung betrifft ein Bleichsystem enthaltend mindestens eine Komponente ausgewählt aus Bleichmittel, Bleichaktivator oder Bleichkatalysator, wobei das Bleichsystem mit mindestens einem Polymer schichtförmig umhüllt ist. Weiterhin betrifft die vorliegende Erfindung ein Verfahren zur Herstellung dieses Bleichsystems eine Reinigerformulierung enthaltend dieses Bleichsystem sowie die Verwendung dieses Bleichsystems beziehungsweise der Reinigerformulierung beispielsweise zum Reinigen oder Waschen von Haushaltstextilien oder von Geschirr.

Reinigerformulierungen (Reinigungsmittel), die zum Reinigen oder Waschen von beispielsweise Textilien oder Geschirr verwendet werden, werden in zahlreichen Variationen hinsichtlich ihrer Zusammensetzung angeboten, wobei die jeweilige Zusammensetzung einen besonderen Einfluss auf die Wirkungsweise der speziellen Reinigerformulierung hat. Solche Reinigerformulierungen enthalten generell ein Bleichsystem, das wiederum mindestens eine Komponente ausgewählt aus der Gruppe von Bleichmittel, Bleichaktivator und Bleichkatalysator enthält. Je nach Verwendung können solche Reinigerformulierungen auch mehrere der vorgenannten Komponenten gleichzeitig enthalten, wobei diese sich in ihrer Wirkungsweise gegenseitig beeinflussen können. Da die einzelnen Komponenten eines Bleichsystems teilweise sehr leicht abreagieren können, besteht ein großes Interesse daran, einerseits möglichst stabile Bleichsysteme bereitzustellen, das heißt die Bleichsysteme sollen erst bei ihrer eigentlichen Verwendung, beispielsweise während des Spülvorgangs in einer Geschirrspülmaschine durch chemische Reaktion, den gewünschten Reinigungseffekt erzielen. Unerwünscht ist hingegen, dass einzelne Komponenten des jeweiligen Bleichsystems bereits vorher, beispielsweise infolge von Luftfeuchtigkeit während der Lagerung, zumindest teilweise chemisch abreagieren. Andererseits sollen lagerstabile Bleichsysteme bei der gewünschten Verwendung zum geeigneten Zeitpunkt möglichst vollständig und zeitgenau einsatzbereit sein.

DE-A 103 61 100 betrifft lagerstabile Kapseln auf Basis von Peroxycarbonsäuren, die beispielsweise als Bestandteil von Wasch- und Reinigungsmitteln verwendet werden können. Die darin beschriebenen organischen Mono- oder Diperoxycarbonsäuren sind bekannte Bleichmittel, die von einem anorganischen Salz umhüllt in Kapselform vorliegen. Das anorganische Salz ist ein nicht basisches, vorzugsweise ein neutrales oder ein insbesondere schwach saures Salz, beispielsweise ein Sulfat-, Nitrat- oder Phosphatsalz.

DE-A 103 61 170 beschreibt ein weiteres lagerstabiles Kapselsystem auf Basis von Peroxycarbonsäuren, wobei in diesem Fall die Kapselhülle mehrschichtig ist und die Kapselumhüllung aus mindestens zwei unterschiedlichen, direkt aufeinander folgenden Hüllschichten auf Basis eines Polyelektrolyten und/oder eines ionischen Tensides bestehen. Vorzugsweise enthält die auf den Kapselkern (organische Peroxycarbonsäure) folgende erste Hüllschicht eine positive Nettoladung (kationisches Tensid oder kationischer Polyelektrolyt) und die zweite Hüllschicht eine negative Nettoladung (vorzugsweise anionischer Polyelektrolyt). Beispiele für kationische Tenside sind quarternäre Ammoniumsalze, ein kationischer Polyelektrolyt kann ein Aminoxid oder Pyrridin-N-oxid sein und der anionische Polyelektrollyt kann eine polymere Sulfonsäure oder eine Polycarbonsäure sein.

US-B 6,380,146 betrifft eine Bleich-Reinigungsmittel-Zusammensetzung, die ein Tensid, ein Phenol oxidierendes Enzym sowie eine weitere Verbindung enthält. Diese weitere Verbindung wirkt als Bleichmittel und zudem als Verstärker des Phenol oxidierenden Enzyms, das den Hauptbestandteil des Bleichsystems dieser Formulierung bildet.

DE-A 196 45 024 betrifft Bleichhilfsmittel enthaltende Mikrokapseln, die in Wasch- und Reinigungsmitteln enthalten sein können. Die Mikrokapseln sind erhältlich durch Polymerisation eines monomeren Gemisches, enthaltend mindestens ein ethylenisch ungesättigtes Carbonsäureanhydrid (Monomer a)) sowie gegebenenfalls weitere Monomere wie (i) zu den Monomeren a) verschiedene monoethylenisch ungesättigte Monomere, (ii) vernetzend wirkende Monomere, die mindestens zwei monoethylenisch ungesättigte, nicht konjugierende Doppelbindungen im Molekül aufweisen, oder (iii) wasserlösliche monoethylenisch ungesättigte Monomere.

US-A 5,417,982 betrifft Formulierungen, die eine kontrollierte Freisetzung von Arzneimitteln oder Hormonen bewirken, wobei die entsprechenden Arzneimittel oder Hormone in einer Polymermatrix suspendiert sind. Die Polymermatrix wird von zumindest zwei hoch wasserlöslichen, biologisch abbaubaren Polymeren wie beispielsweise Cellulosederivaten oder Stärke gebildet. Die Polymermatrix ist wiederum von einem Copolymer aus Milchsäure und Glykolsäure umhüllt, wobei diese Umhüllung die Polymermatrix widerstandsfähiger gegenüber enzymatischem Abbau macht.

DE-A 197 06 023 betrifft den vollständigen Abbau von Formkörpern, Flächengebilden, Beschichtungen, Verklebungen oder Schäumen aus biologisch abbaubaren Polymeren mit Enzymen, insbesondere wird der enzymatische Abbau von Polyesteramiden und Harnstoffgruppen aufweisende Polyesterurethane beschrieben. Als Enzyme geeignet sind Lipasen ausgewählt aus der Gruppe der Lipase aus Candida antarctica Komponenten B, der Lipase Lipozyme 20.000 L und der Lipase aus Aspergilus niger oder Kombinationen davon mit weiteren Enzymen.

WO 97/43014 betrifft ein weiteres Verfahren zum enzymatischen Abbau von Polyesteramiden, wobei als Enzyme Esterasen, Lipasen und Proteasen wie beispielsweise Bakterien der Gattung Bacillus verwendet werden.

WO 02/095127 betrifft die Verwendung von lipolytischen Enzymen bei der Papierherstellung aus Altpapier. Unter lipolytischen Enzymen werden alle Enzyme verstanden, die ein Polymer hydrolisieren können, das als monomeren Baustein Vinylacetat umfasst, insbesondere solche, die durch die EC-Nummer (enzyme commission number) EC 3.1.1.X klassifiziert sind.

WO 01/14629 betrifft ein Verfahren zur enzymatischen Modifizierung der Eigenschaften von Polyesterfasern, wobei polyesterhaltige Artikel mit einer Polyesterase behandelt werden, so dass die Fähigkeit dieser Polyesterartikel, Verbindungen auf deren Oberfläche kovalent oder nicht kovalent zu binden, verbessert wird.

EP-A 1 264 812 betrifft Düngemittel, die mit wenigstens einer biologisch abbaubaren Polymerschicht beschichtet sind, wobei die Polymerschicht hergestellt wird, indem Dispersionen, enthaltend Polymere, die Urethan- und Harnstoffgruppen (also spezielle Polyurethane) aufweisen, auf die Düngemittel aufgebracht werden. Diese mit Polyurethanen umschichteten Düngemittel werden in den Boden gebracht, wo die Nährstoffe über einen längeren Zeitraum hinweg freigesetzt werden, in dem sich die Polymerschicht langsam zersetzt.

Die Aufgabe der vorliegenden Erfindung besteht in der Bereitstellung eines Bleichsystems bzw. von Reinigerformulierungen, die dieses Bleichsystem enthalten, wobei das Bleichsystem lagerstabil sein soll.

Erfindungsgemäß wird diese Aufgabe gelöst durch ein Bleichsystem enthaltend mindestens eine Komponente ausgewählt aus Bleichmittel, Bleichaktivator oder Bleichkatalysator, wobei das Bleichsystem mit mindestens einem Polymer schichtförmig umhüllt ist und das Polymer Urethan- und Harnstoffgruppen aufweist.

Ein Vorteil der erfindungsgemäßen Bleichsysteme ist darin zu sehen, dass sie aufgrund der Umhüllung sehr lagerstabil sind. So tritt beispielsweise kein Verlust an Bleichsystem durch Luftfeuchtigkeit auf, da die das Bleichsystem schichtförmig umhüllenden Polymere bei Trocknung wasserunlöslich werden. Diese Wasserunlöslichkeit kann vorzugsweise dadurch eingestellt werden, indem die flüchtige basische Aminkomponente, beispielsweise Ammoniak, während des Trocknungsprozesses aus der Polymerbeschichtung entfernt wird. Eine Auflösung der Polymerschicht kann in Abhängigkeit des Trocknungsgrads so eingestellt werden, dass die erfindungsgemäßen Bleichsysteme auch in gängigen wasserhaltigen Reinigerkonzentraten eingesetzt werden können. Bei erhöhter Temperatur und hoher Verdünnung mit Wasser kann die Polymerhülle des erfindungsgemäßen Bleichsystems aufgelöst werden, so dass die einzelnen Komponenten des vormals umhüllten Bleichsystems den gewünschte Reinigungs- oder Wascheffekt bewirken können. Sofern das erfindungsgemäße Bleichsystem in Reinigerformulierungen verwendet wird, die zusätzlich Enzyme aufweisen, ist eine schnellere Auflösung der Polymerhülle und somit eine schnellere Freisetzung der einzelnen Komponenten des Bleichsystems festzustellen. Bei Raumtemperatur bzw. darunter sind die erfindungsgemäßen Bleichsysteme auch in Reinigerformulierungen, die Enzyme und Wasser, vorzugsweise nicht mehr als 10 Gew.-% Wasser, enthalten, lagerstabil.

Ein weiterer Vorteil der erfindungsgemäßen Bleichsysteme ist darin zu sehen, dass es prinzipiell ausreichend ist, wenn die einzelnen Komponenten des Bleichsystems mit einem Polymer schichtförmig umhüllt sind. Im Gegensatz dazu sind im Stand der Technik auch Bleichsysteme beschrieben, bei denen einzelne Komponenten mit mindestens zwei oder mehr unterschiedlichen Polymerschichten umhüllt sind. Gegebenenfalls können die erfindungsgemäßen Bleichsysteme aber auch mit zwei oder mehreren Polymeren schichtförmig umhüllt sein. Gegebenenfalls kann die Umhüllung auch aus mehreren Schichten von zwei unterschiedlichen Polymeren bestehen, wobei sich die einzelnen Polymerschichten alternierend abwechseln.

Ein weiterer Vorteil der erfindungsgemäßen Bleichsysteme ist darin zu sehen, dass es prinzipiell möglich ist, alle oder nur einzelne Komponenten ausgewählt aus Bleichmittel, Bleichaktivator oder Bleichkatalysator mit dem Polymer schichtförmig zu umhüllen. Je nachdem ob eine schnelle Freisetzung des Bleichsystems oder eine zeit- bzw. temperaturverzögerte Wirkung des Bleichsystems gewünscht ist, können einzelne Komponenten des Bleichsystems auch in nicht umhüllter Form vorliegen. Ebenso ist es denkbar, dass eine Teilmenge der gleichen Bleichsystemkomponente (beispielsweise ein Bleichaktivator) erfindungsgemäß mit einem Polymer schichtförmig umhüllt ist, während die andere Teilmenge der gleichen Substanz in nicht umhüllter Form in der entsprechenden Reinigerformulierung vorhanden ist.

Im Rahmen der vorliegenden Erfindung soll unter dem Begriff Bleichsystem mindestens eine Komponente ausgewählt aus Bleichmittel, Bleichaktivator oder Bleichkatalysator verstanden werden. Bleichmittel, Bleichaktivator und Bleichkatalysator sind im Rahmen der vorliegenden Erfindung wie folgt definiert:

### Bleichmittel

Als Bleichmittel eignen sich: Sauerstoffbleichmittel wie organische Persäuren, beispielsweise Perbenzoesäure, Peroxy-alpha-Napthoesäure, Peroxylaurinsäure, Peroxystearinsäure, Phthalimidoperoxycapronsäure, 6-Phthalimidoperoxyhexansäure (PAP), Nonylimidperoxybernsteinsäure, Nonylimidperoxyadipinsäure, 1,12-Diperoxydodecandisäure, 1,9-Diperoxyazelainsäure, Diperoxoisophthalsäure und 2-Decyl-diperoxybutan-1,4-disäure. Weiterhin eignen sich kationische Peroxysäuren, wie sie in US-A 5,422,028, US-A 5,294,362 und US-A 5,292,447 beschrieben sind, sowie Sulfonylperoxysäuren, wie sie beispielsweise in US-A 5,039,447 beschrieben sind. Weiterhin kann der Zusatz geringer Mengen von Bleichmittelstabilisatoren wie beispielsweise Phosphonaten, Boraten, Metaboraten, Metasilikaten und Magnesiumsalzen sinnvoll sein.

Bevorzugte Bleichmittel sind Perbenzoesäure, Peroxy-alpha-Napthoesäure, Peroxylaurinsäure, Peroxystearinsäure, Phthalimidoperoxycapronsäure, 6-Phthalimidoperoxyhexansäure (PAP), Nonylimidperoxybernsteinsäure, Nonylimidperoxyadipinsäure, 1,12-Diperoxydodecandisäure, 1,9-Diperoxyazelainsäure, Diperoxoisophthalsäure und 2-Decyldiperoxybutan-1,4-disäure.

Besonders bevorzugt als Bleichmittel ist 6-Phthalimidoperoxyhexansäure (PAP).

### Bleichaktivator

Bleichaktivatoren sind beispielsweise Verbindungen, die unter Perhydrolysebedingungen aliphatische Peroxocarbonsäuren mit vorzugsweise 1 bis 10 C-Atomen, insbesondere 2 bis 4 C-Atömen, und/oder gegebenenfalls substituierte Perbenzoesäure ergeben. Dabei handelt es sich häufig um Bleichaktivatoren, die eine oder mehrere N- bzw. O-Acylgruppen enthalten und/oder gegebenenfalls substituierte Benzoylgruppen tragen, wie Substanzen aus der Klasse der Anhydride, der Ester, der Imide und der acylierten Imidazole oder Oxime. Beispiele dafür sind Tetraacetylethylendiamin (TAED), Tetraacetylmethylendiamin (TAMD), Tetraacetylglykoluril (TAGU), Tetraacetylhexylendiamin (TAHD), N-Acylimide, insbesondere N-Nonanoylsuccinimid (NOSI), acylierte Phenolsulfonate, insbesondere n-Nonanoyl- oder lsononanoyloxybenzolsulfonate (n- bzw. iso-NOBS) und Lauroyloxybenzolsulfonate (LOBS), Pentaacetylglucose (PAG), 1,5-Diacetyl-2,2-dioxo-hexahydro-1,3,5-triazin (DADHT) und Isatosäureanhydrid (ISA).

Weiterhin geeignet sind Bleichaktivatoren aus der Gruppe der Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und die aus den deutschen Patentanmeldungen DE-A 196 16 693 und DE-A 196 16 767 bekannten Enolester sowie acetyliertes Sorbitol und Mannitol bzw. deren in der europäischen Patentanmeldung EP-A 0 525 239 beschriebene Mischungen (SORMAN), acylierte Zuckerderivate, insbesondere Pentaacetylglukose (PAG), Pentaacetylfructose, Tetraacetylxylose und Octaacetyllactose, sowie acetyliertes, gegebenenfalls N-alkyliertes Glucamin und Gluconolacton, und/oder N-acylierte Lactame, beispielsweise N-Benzoylcaprolactam und Carbonylbiscaprolactam, die aus WO 94/27970, WO 94/28102, WO 94/28103, WO 95/00626, WO 95/14759, WO 95/17498 und WO 96/36686 bekannt sind, weiterhin auch Bis(2-Propylimino)carbonat, siehe DE-A 195 18 039, DE-A 195 41 012, DE-A 196 09 953 und DE-A 197 04 149. Weiterhin eignen sich die aus DE-A 196 16 769 bekannten hydrophil substituierten Acylacetale und die in DE-A 196 16 770 sowie WO 95/14075 beschriebenen Acyllactame.

Das Bleichmittel der vorliegenden Erfindung kann auch in Kombination mit sogenannten Bleichkraftverstärkern eingesetzt werden. Dabei handelt es sich um Substanzen, die die Wirkung der bekannten Bleichmittel noch weiter steigern. Als Bleichaktivatoren eignen sich insbesondere die Diamine, die in DE-A 196 11 992 beschrieben sind. Dabei handelt es sich um Verbindungen, die sekundäre Amingruppen-NHR¹ enthalten und die niedermolekular, oligomer oder polymer sind. Insbesondere handelt es sich um sekundäre Amine der allgemeinen Formel (I)

R¹NH-[(CR³R⁴)ₘ-NH]ₙ-R² (I),

wobei n einen ganzzahligen Wert von 0 bis 20 und m einen ganzzahligen Wert von 2 bis 4 hat, die Reste R³ und R⁴ unabhängig C₁-C₃₀-, vorzugsweise C₁-C₁₅-Alkylreste sind und die Reste R¹ und R² unabhängig voneinander C₁-C₃₀-, vorzugsweise C₁-C₁₅-Alkylreste sind oder gegebenenfalls gemeinsam einen Zyklus ausbilden.

### Bleichkatalysator

Zusätzlich zu den oben aufgeführten konventionellen Bleichaktivatoren oder an deren Stelle können auch die aus EP-A 0 446 982 und EP-A 0 453 003 bekannten Sulfonimine und/oder bleichverstärkende Übergangsmetallsalze beziehungsweise Übergangsmetallkomplexe als sogenannte Bleichkatalysatoren in den erfindungsgemäßen Reinigerformulierungen enthalten sein. Zu den in Frage kommenden Übergangsmetallverbindungen gehören insbesondere die aus DE-A 195 29 905 bekannten Mangan-, Eisen-, Cobalt-, Ruthenium- oder Molydän-Salenkomplexe und deren aus DE-A 196 20 267 bekannte N-Analogverbindungen, die aus DE-A 195 36 082 bekannten Mangan-, Eisen-, Cobalt-, Ruthenium- oder Molybdän-Carbonylkomplexe, die in DE-A 196 05 688 beschriebenen Mangan-, Eisen-, Cobalt, Ruthenium-, Molybdän-, Titan-, Vanadium- und Kupfer-Komplexe mit stickstoffhaltigen Tripod-Liganden, die aus DE-A 196 20 411 bekannten Cobalt-, Eisen-, Kupfer- und Ruthenium-Amminkomplexe, die in DE-A 44 16 438 beschriebenen Mangan-, Kupfer- und Cobalt-Komplexe, die in EP-A 0 272 030 beschriebenen Cobalt-Komplexe, die aus EP-A 0 693 550 bekannten Mangan-Komplexe, die aus EP-A 0 392 592 bekannten Mangan-, Eisen-, Cobalt- und Kupfer-Komplexe und/oder die in der europäischen Patentschrift EP-B 0 443 651, EP-A 0458 397, EP-A 0458 398, EP-A 0549 271, EP-A 0 549 272, EP-A 0 544 490 oder EP-A 0 544 519 beschriebenen Mangan-Komplexe.

Im Rahmen der vorliegenden Erfindung können bleichverstärkende Übergangsmetallkomplexe, insbesondere mit den Zentralatomen Mn, Fe, Co, Cu, Mo, V, Ti und/oder Ru, bevorzugt ausgewählt aus der Gruppe der Mangan- und Cobaltsalze und -komplexe, besonders bevorzugt der Cobalt(ammin)-Komplexe, der Cobalt(acetat)-Komplexe, der Cobalt(carbonyl)-Komplexe, der Chloride des Cobalts und Mangans und des Mangansulfats ausgewählt werden.

Das erfindungsgemäße Bleichsystem bzw. seine einzelnen, vorgenannten Komponenten sind vorzugsweise fest und weiterhin vorzugsweise partikelförmig. Erfindungsgemäß ist im Bleichsystem mindestens eine Komponente ausgewählt aus Bleichmittel, Bleichaktivator oder Bleichkatalysator mit mindestens einem Polymer schichtförmig umhüllt, wobei das Polymer Urethan- und Harnstoffgruppen aufweist. Gegebenenfalls können auch alle Komponenten des Bleichsystems mit mindestens einem Polymer schichtförmig umhüllt sein. In einer bevorzugten Ausführungsform ist nur ein Bleichmittel von mindestens einem Polymer schichtförmig umhüllt. Gegebenenfalls können die einzelnen Komponenten des Bleichmittelsystems getrennt voneinander mit mindestens einem Polymer schichtförmig umhüllt werden oder sie können ganz oder teilweise als Gemisch von mindestens einem Polymer schichtförmig umhüllt sein.

### Polymer

Als Polymere, die Harnstoff und Urethangruppen aufweisen, kommen erfindungsgemäß vorzugsweise solche in Betracht, die in EP-A 1 264 812 beschrieben sind.

Bevorzugt sind dies Polymere auf der Basis von Polyesterpolyolen und Isocyanaten. Mehr bevorzugt sind die Polymere auf der Basis aliphatischer Isocyanate. Besonders bevorzugt sind Polymere, die erhältlich sind, indem
a) aus Makroolen, ionischen oder potentiell ionischen Polyolen und überschüssigen Polyisocyanaten ein NCO-teminiertes Prepolymer hergestellt wird,
b) dieses Prepolymer mit Verbindungen, die mindestens 2 gegenüber Isocyanat reaktive Aminogruppen aufweisen, in einem Verhältnis NCO-Gruppen (bezogen auf das Prepolymer)/NH-Gruppen (bezogen auf die reaktiven Aminogruppen) von ≤ 1:1 umgesetzt und
c) neutralisiert wird.

Als Makroole werden solche Verbindungen eingesetzt, die ein Molekulargewicht von 500 bis 5000, vorzugsweise von 800 bis 4500, höchst bevorzugt von 800 bis 3000 aufweisen. Besonders bevorzugt ist der Einsatz von Makrodiolen.

Bei den Makroolen handelt es sich insbesondere um Polyesterpolyole, die zum Beispiel aus Ullmanns Encyklopädie der technischen Chemie, 4. Auflage, Band 19, S. 62-65 bekannt sind. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, araliphatisch, aromatisch oder heterocyclisch sein und gegebenenfalls, zum Beispiel durch Halogenatome, substituiert und/oder ungesättigt sein. Als Beispiele hierfür seien genannt: Korksäure, Azelainsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Alkenylbernsteinsäure, Fumarsäure, dimere Fettsäuren. Bevorzugt sind Dicarbonsäuren der allgemeinen Formel HOOC-(CH₂)y-COOH, wobei y eine Zahl von 1 bis 20, bevorzugt eine gerade Zahl von 2 bis 20 ist, z.B. Bernsteinsäure, Adipinsäure, Dodecandicarbonsäure und Sebacinsäure.

Als Diole kommen z.B. Ethylenglykol, Propan-1,2-diol, Propan-1,3-diol, Butan-1,3-diol, Butan-1,4-diol, Buten-1,4-diol, Butin-1,4-diol, Pentan-1,5-diol, Hexan-1,6-diol, Neopentylglykol, Bis-(hydroxymethyl)-cyclohexane wie 1,4-Bis-(hydroxymethyl)cyclohexan, 2-Methylpropan-1,3-diol, Methylpentandiole, ferner Dipropylenglykol, Polypropylenglykol, Dibutylenglykol und Polybutylenglykole in Betracht. Bevorzugt sind Alkohole der allgemeinen Formel HO-(CH₂)ₓ-dH, wobei x eine Zahl von 1 bis 20, bevorzugt eine gerade Zahl von 2 bis 20 ist. Beispiele hierfür sind Ethylenglykol, Butan-1,4-diol, Hexan-1 ,6-diol, Octan-1,8-diol und Dodecan-1,12-diol. Weiterhin bevorzugt sind Neopentylglykol und Pentandiol-1,5.

Ferner kommen auch Polycarbonat-Diole, wie sie zum Beispiel durch Umsetzung von Phosgen mit einem Überschuss von den als Aufbaukomponenten für die Polyesterpolyole genannten niedermolekularen Alkohole erhalten werden können, in Betracht.

Geeignet sind auch Polyesterdiole auf Lacton-Basis, wobei es sich um Homo- oder Mischpolymerisate von Lactonen, bevorzugt um endständige Hydroxylgruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁-C₄-Alkylrest substituiert sein kann. Beispiele sind ε-Caprolacton, β-Propiolacton, γ-Butyrolacton und/oder Methyl-ε-caprolacton sowie deren Gemische. Geeignete Starterkomponenten sind zum Beispiel die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die chemisch äquivalenten Polykondensate der den Lactonen entsprechenden Hydroxycarbonsäuren, eingesetzt werden.

Daneben kommen als Monomere Polyetherole in Betracht. Sie sind insbesondere durch Polymerisation von Propylenoxid, Butylenoxid, Tetrahydrofuran, Styroloxid oder Epichlorhydrin mit sich selbst, zum Beispiel in Gegenwart von BF₃ oder durch Anlagerung dieser Verbindungen gegebenenfalls im Gemisch oder nacheinander, an Startkomponenten mit reaktionsfähigen Wasserstoffatomen, wie Alkohole oder Amine, zum Beispiel Wasser, Ethylenglykol, Propan-1,2-diol, 1,2-Bis-(4-hydroxyphenyl)-propan oder Anilin erhältlich. Besonders bevorzugt ist Polytetrahydrofuran eines Molekulargewichts von 240 bis 5000, und vor allem 500 bis 4500.

Ebenfalls geeignet sind Polyhydroxyolefine, bevorzugt solche mit 2 endständigen Hydroxylgruppen, zum Beispiel α-ω-Dihydroxypolybutadien, α-ωDihydroxypolymethacrylester oder α-ω-Dihydroxypolyacrylester als Monomere. Solche Verbindungen sind beispielsweise aus EP-A-0 622 378 bekannt. Weitere geeignete Polyole sind Polyacetale, Polysiloxane und Alkydharze.

Neben den genannten Makroolen können gegebenenfalls auch kurzkettige Polyole zugesetzt werden. Hierbei kommen beispielsweise kurzkettige Diole mit einem Molekulargewicht von 62 bis 500, insbesondere 62 bis 200 g/mol in Betracht.

Als kurzkettige Diole werden vor allem als Aufbaukomponenten die für die Herstellung von Polyesterpolyolen genannten kurzkettigen Alkandiole eingesetzt, wobei die unverzweigte Diole mit 2 bis 12 C-Atomen und einer gradzahligen Anzahl von C-Atomen sowie Pentan-1,5-diol bevorzugt werden. Außerdem kommen Phenole, aromatische DihydroxyVerbindungen oder Bisphenol A oder F als Diole in Betracht.

Als ionische oder potentiell ionische Polyole kommen erfindungsgemäß 2,2-Di-(hydroxymethyl)-Alkan-Monocarbonsäuren mit insgesamt bis zu 10 Kohlenstoffatomen in Betracht. Als Monomere mit (potentiell) anionischen Gruppen kommen üblicherweise aliphatische, cycloaliphatische, araliphatische oder aromatische Carbonsäuren und Sulfonsäuren in Betracht, die mindestens eine alkoholische Hydroxylgruppe oder mindestens eine primäre oder sekundäre Aminogruppe tragen, vor allem, mit 3 bis 10 Kohlenstoffaromen, wie sie auch in US-A 3 412 054 beschrieben sind. Insbesondere sind Verbindungen der allgemeinen Formel (II) 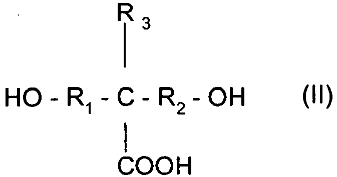 in welcher R¹ und R² für eine C₁-C₄- Alkandiyl-Einheit und R³ für eine C₁-C₄-Alkyl-Einheit steht geeignet. Besonders bevorzugt ist Dimethylolpropionsäure (DMPA).

Als Polyisocyanate kommen erfindungsgemäß vorzugsweise die in der Polyurethanchemie üblicherweise eingesetzten Diisocyanate in Betracht.

Insbesondere zu nennen sind Diisocyanate X(NCO)₂ wobei X für einen aliphatischen Kohlenwasserstoffrest mit 4 bis 12 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht. Beispiele derartiger Diisocyanate sind Tetramethylendiisocyanat, Hexamethylendiisocyanat, Dodecamethylendiisocyanat, 1,4-Diisocyanatocylohexan, 1-Isocyanato-3,5,5-trimethyl-5-isocyanatomethylcyclohexan (IPDI), 2,2-Bis-(4-isocyanatocyclohexyl)-propan, Trimethylhexandiisocyanat, 1,4-Diisocyanatobenzol, 2,4-Diisocyanatotoluol, 2,6-Diisocyanatotoluol, 4,4-Diisocyanato-diphenylmethan, 2,4-Diisocyanato-diphenylmethan, p-Xyxlylendiisocyanat, Tetramethylxylylendiisocyanat (TMXDI), die Isomeren des Bis-(4-isocyanatocyclohexyl)methans (HMDI) wie das trans/trans-, das cis/cis- und das cis/trans-Isomer sowie aus diesen Verbindungen bestehende Gemische.

Als Gemische dieser Isocyanate sind besonders die Mischungen der jeweiligen Strukturisomeren von Diisocyanatotoluol und Diisocyanato-diphenylmethan von Bedeutung, insbesondere ist die Mischung aus 80 mol-% 2,4 Diisocyanatotoluol und 20 mol-% 2,6-Diisocyanatotoluol geeignet. Weiterhin sind die Mischungen von aromatischen Isocyanaten wie 2,4-Diisocyanatotoluol und/oder 2,6-Diisocyanatotoluol mit aliphatischen oder cycloaliphatischen Isocyanaten wie Hexamethylendiisocyanat oder IPDI besonders vorteilhaft, wobei das bevorzugte Mischungsverhältnis der aliphatischen zu aromatischen Isocyanate 4:1 bis 1:4 beträgt. Ganz besonders bevorzugt werden nur Isocyanate verwendet, die ausschließlich aliphatisch gebundene NCO-Gruppen tragen.

Als Polyisocyanate kann man auch Isocyanate einsetzen, die neben freien NCO-Gruppen weitere, von NCO-Gruppen abgeleitete Gruppen, wie zum Beispiel Isocyanurat-, Biuret-, Harnstoff-, Allophanat-, Uretdion- oder Carbodiimidgruppen tragen.

Die beschriebenen Makroole, ionischen oder potentiell ionischen Polyole sowie Isocyanate und gegebenenfalls kurzkettigen Polyole werden zu einem NCO-terminierten Prepolymer umgesetzt. Die Umsetzung kann gegebenenfalls in einem inerten Lösungsmittel durchgeführt werden, beispielsweise Aceton, Methylethylketon, Diethylketon oder Essigsäureethylester. Hierbei werden vorzugsweise difunktionelle Bausteine enthaltende Polyole verwendet. Das Verhältnis von NCO-Gruppen zu NCO-reaktiven Gruppen soll erfindungsgemäß zwischen 1,1:1 und 2:1, vorzugsweise zwischen 1,15:1 und 1,9:1, besonders bevorzugt zwischen 1,2:1 und 1,5:1 liegen.

In vorteilhafter Weise wird erfindungsgemäß ein Überschuss an NCO eingesetzt.

Dieses Prepolymer wird im Schritt b weiter umgesetzt. Als Reaktionskomponente können alle aliphatischen und/oder cycloaliphatischen Verbindungen verwendet werden, die mindestens zwei gegenüber Isocyanaten reaktive Aminogruppen tragen. Bevorzugt ist der Einsatz von Diamin. Hierfür in Frage kommen insbesondere Ethylendiamin, Propylendiamin, Hexamethylendiamin, Isophorondiamin (IPDA), p-Xylylendiamin, 4,4-Diaminodicyclohexylmethan und 4,4-Diamino-3,3-dimethyldicyclohexylmethan.

Das Prepolymer wird mit den genannten Verbindungen vorzugsweise in einem NCO-Gruppen/NH-Gruppen-Verhältnis von 0,9:1 bis 1:1 umgesetzt. Besonders bevorzugt ist erfindungsgemäß ein Verhältnis von 0,95:1 bis 1:1, ganz besonders 1:1. Daraus folgt, dass der NCO-Gehalt nach Schritt b) 0, maximal 0,2 Gew.-% bezogen auf das Prepolymer ist.

Im Anschluss an die Umsetzung des Prepolymers erfolgt eine Neutralisierung. Hierfür sind z.B. Ammoniak, N-Methylmorpholin, Dimethylisopropanolamin, Triethylamin, Diemthylethanolamin, Methyldiethanolamin, Triethanolamin, Morpholin, Tripropylamin, Ethanolamin, Diethanolamin, Triisopropanolamin, N-Ethyldiisopropylamin und Gemische daraus geeignet.

Besonders bevorzugt ist erfindungsgemäß der Einsatz von Ammoniak. Der Gehalt an Ammoniumcarboxylatgruppen (COO⁻ NH₄⁺), die durch die Neutralisierung erhalten werden, soll erfindungsgemäß zwischen 100 und 600 mmol/kg, vorzugsweise 200 bis 500, besonders bevorzugt 250 bis 500 liegen.

Das erfindungsgemäße Bleichsystem, das mit mindestens einem Polymer schichtförmig umhüllt ist, ist vorzugsweise partikelförmig. Sofern die einzelnen Komponenten des Bleichsystems partikelförmig sind, bedeutet dies somit, dass die einzelnen Partikel jeweils eine separate Polymerumhüllung aufweisen. Unter Partikel soll gegebenenfalls auch ein Agglomerat von einzelnen Partikeln verstanden werden. Der mittlere Partikeldurchmesser einzelner polymerumhüllter Bleichsystempartikel ist vorzugsweise 0,01 bis 5 mm, mehr bevorzugt 0,1bis 3 mm, noch mehr bevorzugt 0,7 bis 2,5 mm, besonders bevorzugt 1,2 bis 2,5 mm.

Die Schichtdicke eines einzelnen, das Bleichsystem umhüllenden Polymers ist vorzugsweise von 10 bis 2000 µm, mehr bevorzugt 10 bis 1500 µm und besonders bevorzugt 20 bis 800 µm. Sofern das Bleichsystem mit mehreren Polymerschichten umhüllt ist, weisen die einzelnen Polymerschichten unabhängig voneinander die vorgenannten Schichtdicken auf.

Ein weiterer Gegenstand der vorliegenden Erfindung ist ein Verfahren zur Herstellung des erfindungsgemäßen Bleichsystems. Das erfindungsgemäße Verfahren wird durchgeführt, indem eine Dispersion, vorzugsweise eine Suspension, enthaltend Polymere, die Urethan-und Harnstoffgruppen aufweisen, auf das Bleichsystem aufgebracht wird. Wie vorstehend bereits aufgeführt, sind die Polymere sowie deren Herstellverfahren bereits bekannt. Vorzugsweise werden wässrige Dispersionen enthaltend die vorgenannten Polymere eingesetzt, wobei der Polymergehalt der Dispersion vorzugsweise 10 bis 65 Gew.-%, mehr bevorzugt 10 bis 50 Gew.-% an Polymer beträgt. Gegebenenfalls können noch zusätzliche Substanzen in der Dispersion vorliegen, die anschließend gemeinsam mit dem Polymer auf das Bleichsystem aufgebracht werden.

Verfahren zum Aufbringen von Dispersionen enthaltend Polymere auf Bleichsysteme sind dem Fachmann bekannt. Vorzugsweise erfolgt das Aufbringen der Dispersionen zweckmäßigerweise durch Aufsprühen. Die erfindungsgemäß verwendeten Dispersionen können für Beschichtungsverfahren bei erhöhter Prozesstemperatur eingesetzt werden. Die bei höheren Temperaturen wesentlich höheren Wasserdampfdrücke steigern die Kapazität einer Beschichtungsanlage erheblich. Vorzugsweise erfolgt das Beschichten bei einer Temperatur 10 bis 110 °C, vorzugsweise 30 bis 70 °C.

Um zu vermeiden, dass das erfindungsgemäße Mittel beim Aufbringen der wässrigen Dispersion angelöst wird, bringt man pro Zeiteinheit nur eine begrenzte Menge der Dispersionen auf und sorgt dafür, dass das Wasser-Ammoniak-Gemisch rasch verdunsten kann.

Dies wird zweckmäßigerweise dadurch erreicht, dass man eine Wirbelschicht, die durch Aufwirbeln des Ausgangsdüngemittelgranulats mit einem Wirbelgas erzeugt wird, bei einer Temperatur von 10 bis 110 °C, vorzugsweise 30 bis 70 °C, mit den Dispersionen besprüht. Nach dem Aufsprühen der Lösungen bzw. Dispersionen wird die Wirbelschicht solange aufrechterhalten, bis das Dispergiermedium verdunstet ist.

Derartige Wirbelschicht-Auftragsverfahren sind allgemein bekannt und für die Herstellung von beschichteten Düngemittelgranulaten in der US-A 5,211,985 beschrieben. Mit diesem Verfahren lassen sich besonders gleichmäßige und dünne Beschichtungen erzeugen, die im allgemeinen eine Dicke von ungefähr 10 bis ungefähr 1500 µm, vorzugsweise ungefähr 10 bis ungefähr 1000 µm und insbesondere ungefähr 20 bis ungefähr 800 µm aufweisen.

Erfindungsgemäß können auf die Mittel eine oder mehrere Schichten aufgebracht werden. In einer Variante der Erfindung werden auf das Mittel mindestens eine innere Schicht und eine äußere Schicht aufgebracht, wobei vorzugsweise die äußere Schicht aus einer Dispersion hergestellt wird, die die beschriebenen Polyharnstoff-Polyurethane enthält. Als innere Schicht(en) können in einer Variante der Erfindung prinzipiell alle für Beschichtungen von Bleichsysteme verwendbaren Substanzen, die verschieden von den erfindungsgemäß verwendeten Polyharnstoff-Polyurethan-Dispersion sind, eingesetzt werden. Vorzugsweise enthält die innere Schicht mindestens eine biologisch abbaubare Substanz, die jedoch verschieden von der in der äußeren Schicht eingesetzten Polyharnstoff- Polyurethan- Dispersion ist.

In einer bevorzugten Ausführungsform des erfindungsgemäßen Verfahrens werden Polymerdispersionen eingesetzt, die erhältlich sind, indem
a) aus Makroolen, ionischen oder potentiell ionischen Polyolen und überschüssigen Polyisocyanaten ein NCO-terminiertes Präpolymer hergestellt wird,
b) dieses Prepolymer mit Verbindungen, die mindestens 2 gegenüber Isocyanat reaktive Aminogruppen aufweisen, in einem Verhältnis NCO-Gruppen/NH-Gruppen von ≤ 1 : 1 umgesetzt und
c) neutralisiert wird.

Sofern in Schritt a) die Herstellung des Prepolymers in einem Lösungsmittel erfolgt ist, kann dieses Lösungsmittel im Anschluss an den Neutralisationsschritt c) vorzugsweise abdestilliert werden. Als Lösungsmittel eignet sich beispielsweise Aceton, Methylethylketon (MEK) oder Essigsäureethylester

Ein weiterer Gegenstand der vorliegenden Erfindung sind Reinigerformulierungen (Reinigungsmittelzusammensetzungen), die mindestens eines der vorgenannten Bleichsysteme enthalten.

Im Rahmen der vorliegenden Erfindung können in diesen Reinigerformulierungen auch einzelne oder mehrere Komponenten des Bleichsystems in nicht umhüllter Form vorliegen und einzelne Komponenten können mit einem Polymer in schichtförmig umhüllter Form vorliegen, wobei das Polymer Urethan- und Harnstoffgruppen aufweist. Einzige Voraussetzung ist, dass mindestens eine Komponente ausgewählt aus Bleichmittel, Bleichaktivator oder Bleichkatalysator mit mindestens einem Polymer schichtförmig umhüllt ist und das Polymer Urethan- und Harnstoffgruppen aufweist.

In einer bevorzugten Ausführungsform enthalten die erfindungsgemäßen Reinigerformulierungen ein erfindungsgemäßes (umhülltes) Bleichsystem, das mindestens ein Bleichmittel, aber keinen Bleichaktivator und keinen Bleichkatalysator enthält. Bleichaktivator und Bleichkatalysator können jedoch in nicht-umhüllter Form in der erfindungsgemäßen Reinigerformulierung enthalten sein.

Die Reinigerformulierungen können dabei je nach Anwendungsgebiet flüssig, gelförmig, pulverförmig, granulär oder tablettenförmig sein. Gegebenenfalls können sie auch als feste Waschstücke vorliegen. Die Reinigerformulierungen sind dabei je nach ihrer vorgesehenen Anwendung in ihrer Zusammensetzung an den gewünschten Verwendungszweck anzupassen. Dem Fachmann sind gängige Reinigerformulierungen beispielsweise für Textilien oder für Geschirrspülmittel bekannt. Sofern nicht anders aufgeführt, enthalten die erfindungsgemäßen Reinigerformulierungen konventionelle Waschmittelinhaltsstoffe, die dem Stand der Technik entsprechen. Repräsentative Beispiele für solche zusätzlichen Inhaltsstoffe werden nachfolgend beschrieben.

Gegebenenfalls kann in den erfindungsgemäßen Reinigerformulierungen das erfindungsgemäße Bleichsystem mit anderen Formulierungsbestandteilen in bestimmte Kompartimente der Reinigerformulierungen eingearbeitet sein, wobei im Fall von tablettenförmigen Reinigerformulierungen die Kompartimente bestimmte Tablettenschichten und/oder in die Tablette eingelassene, mit der Tablette verklebte oder die Tablette umhüllende Formkörper sein können.

Das Bleichsystem ist in den erfindungsgemäßen Reinigerformulierungen zu 0,1 bis 95 Gew.-% vorhanden. Die Gew.-%-Angaben beziehen sich auf das Gesamtgewicht der Reinigerformulierungen

Die einzelnen Komponenten des Bleichsystems sind, sofern sie in den erfindungsgemäßen Reinigerformulierungen vorhanden sind und dabei mit mindestens einem Polymer schichtförmig umhüllt sind, wobei das Polymer Urethan- und Harnstoffgruppen aufweist, in den folgenden Mengen vorhanden.

Das Bleichmittel wird in den erfindungsgemäßen Reinigerformulierungen vorzugsweise in Mengen bis 95 Gew.-%, insbesondere 0,1 Gew.-% bis 80 Gew.-%, besonders 0,5 bis 80 Gew.-% und besonders bevorzugt 0,8 bis 75 Gew.-% bezogen auf die gesamte Formulierung eingesetzt.

Bleichaktivatoren werden in Mengen von 0,1 bis 20 Gew.-%, bevorzugt von 1 bis 15 Gew.-%, besonders bevorzugt von 1,5 bis 10 Gew.-%, bezogen auf das Gesamtgewicht der Reinigerformulierung, eingesetzt.

Sofern Bleichkatalysatoren, insbesondere bleichverstärkende Übergangsmetallkomplexe vorhanden sind, können diese in üblichen Mengen, vorzugsweise in einer Menge bis zu 5 Gew.-%, insbesondere von 0,0025 Gew.-% bis 1 Gew.-% und besonders bevorzugt von 0,01 Gew.-% bis 0,25 Gew.-%, jeweils bezogen auf die gesamte Reinigerformulierung eingesetzt werden.

Weitere Reinigerformulierungskomponenten, die neben dem erfindungsgemäßen Bleichsystem in den Reinigerformulierungen der vorliegenden Erfindung vorliegen können, sind nachfolgend definiert. Die jeweiligen Konzentrationsangaben beziehen sich auf alle Ausführungsbeispiele, in denen diese optionalen Komponenten enthalten sind.

### Tenside

Prinzipiell können alle dem Fachmann bekannten Tenside in den erfindungsgemäßen Reinigerformulierungen verwendet werden. Vorzugsweise lassen sich jedoch die Tenside in zwei Hauptanwendungsgebiete unterscheiden.

### a) Tenside für Haushaltstextilwaschmittel

Die Gesamtkonzentration von Tensiden in der fertigen Waschmittelformulierung kann von 0,1 bis 99 Gew.-%, bevorzugt von 5 bis 80 Gew.-% betragen. Die verwendeten Tenside können anionisch, nichtionisch, amphoter oder kationisch sein. Es können auch Mischungen der genannten Tenside verwendet werden. Bevorzugte Waschmittelformulierungen enthalten anionische und/oder nichtionische Tenside und deren Mischungen mit weiteren Tensiden.

Als anionische Tenside kommen Sulfate, Sulfonate, Carboxylate, Phosphate und deren Mischungen in Betracht. Geeignete Kationen sind hierbei Alkalimetalle, wie beispielsweise Natrium oder Kalium oder Erdalkalimetalle, wie z. B. Calcium oder Magnesium sowie Ammonium, substituierte Ammoniumverbindungen einschließlich Mono-, Di- oder Triethanolammoniumkationen und Mischungen daraus. Unter den anionischen Tensiden sind Alkylestersulfonate, Alkylsulfate, Alkylethersulfate, Alkylbenzolsulfonate, sekundäre Alkansulfonate und Seifen bevorzugt. Diese werden nachfolgend beschrieben.

Alkylestersulfonate sind unter anderem lineare Ester von C₁₈-C₂₀-Carboxylsäuren (Fettsäuren), welche mittels gasförmigem SO₃ sulfoniert werden, wie dies beispielsweise in "The Journal of the American Oil Chemists Society" 52 (1975), p. 323-329, beschrieben wird. Geeignete Ausgangsmaterialien sind natürliche Fette wie z. B. Talg, Kokosöl und Palmöl, aber auch Fette synthetischer Natur sein. Bevorzugte Alkylestersulfonate sind Verbindungen der Formel (III) 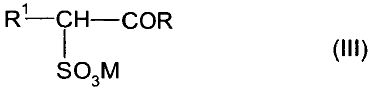 worin R¹ einen C₈-C₂₀-Kohlenwasserstoffrest, bevorzugt Alkyl und R einen C₁-C₆-Kohlenwasserstoffrest, bevorzugt Alkyl, darstellt. M steht für ein Kation, das ein wasserlösliches Salz mit dem Alkylestersulfonat bildet. Geeignete Kationen sind Natrium, Kalium, Lithium oder Ammoniumkationen wie beispielsweise Monoethanolamin, Diethanolamin und Triethanolamin. Bevorzugt bedeuten R¹ C₁₀-C₁₆-Alkyl und R Methyl, Ethyl oder Isopropyl. Meist bevorzugt sind Methylestersulfonate, in denen R¹ C₁₀-C₁₆-Alkyl bedeutet.

Alkylsulfate sind wasserlösliche Salze oder Säuren der Formel ROSO₃M, worin R ein C₁₀-C₂₄-Kohlenwasserstoffrest, bevorzugt ein Alkyl- oder Hydroxyalkylrest mit C₁₀-C₂₀-Alkylkomponente, besonders bevorzugt ein C₁₂-C₁₈-Alkyl- oder Hydroxyalkylrest ist. M ist Wasserstoff oder ein geeignetes Kation, z.B. ein Alkalimetallkation, vorzugsweise Natrium, Kalium, Lithium oder ein Ammonium- oder substituiertes Ammoniumkation, vorzugsweise ein Methyl-, Dimethyl- und Trimethylammoniumkation oder ein quaternäres Ammoniumkationen, wie beispielsweise das Tetramethylammonium- und Dimethylpiperidiniumkationen und von Alkylaminen wie Ethylamin, Diethylamin, Triethylamin und Mischungen davon abgeleitete quartäre Ammoniumkationen. Alkylketten mit C₁₂-C₁₆ sind für niedrige Waschtemperaturen (z. B. unter ca. 50 °C) und Alkylketten mit C₆-C₁₈ für höhere Waschtemperaturen (z. B. oberhalb ca. 50 °C) bevorzugt.

Alkylethersulfate sind wasserlösliche Salze oder Säuren der Formel RO(A)ₘ SO₃M, worin R einen unsubstituierten C₁₀-C₂₄-Alkyl- oder Hydroxyalkylrest, bevorzugt einen C₁₂-C₂₀-Alkyl-oder Hydroxyalkylrest, besonders bevorzugt einen C₁₂-C₁₈-Alkyl- oder Hydroxyalkylrest darstellt. A ist eine Ethoxy- oder Propoxyeinheit, m ist eine Zahl größer als 0, vorzugsweise zwischen ca. 0,5 und ca. 6, besonders bevorzugt zwischen ca. 0,5 und ca. 3 und M ist ein Wasserstoffatom oder ein Kation wie z. B. Natrium, Kalium, Lithium, Calcium, Magnesium, Ammonium oder ein substituiertes Ammoniumkation. Beispiele von substituierten Ammoniumkationen umfassen Methyl-, Dimethyl-, Trimethylammonium- und quaternäre Ammoniumkationen wie Tetramethylammonium und Dimethylpiperidiniumkationen sowie solche, die von Alkylaminen wie Ethylamin, Diethylamin, Triethylamin oder Mischungen davon abgeleitet sind. Als Beispiele seien C₁₂-C₁₈-Fettalkoholethersulfate genannt, wobei der Gehalt an Ethylenoxideinheiten 1, 2, 2,5, 3 oder 4 mol pro mol des Fettalkoholethersulfats beträgt und in denen M Natrium oder Kalium ist.

In sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe kann an einer beliebigen Position der C-Kette sein, wobei die primären Methylgruppen am Kettenanfang und Kettenende keine Sulfonatgruppen besitzen. Die bevorzugten sekundären Alkansulfonate enthalten lineare Alkylketten mit ca. 9 bis 25 Kohlenstoffatomen, bevorzugt ca. 10 bis ca. 20 Kohlenstoffatomen und besonders bevorzugt ca. 13 bis 17 Kohlenstoffatomen. Das Kation ist beispielsweise Natrium, Kalium, Ammonium, Mono-, Di- oder Triethanolammonium, Calcium oder Magnesium und Mischungen davon. Natrium als Kation ist bevorzugt.

Weitere geeignete anionische Tenside sind Alkenyl- oder Alkylbenzolsulfonate. Die Alkenyl- oder Alkylgruppe kann verzweigt oder linear und gegebenenfalls mit einer Hydroxylgruppe substituiert sein. Die bevorzugten Alkylbenzolsulfonate enthalten lineare Alkylketten mit ca. 9 bis 25 Kohlenstoffatomen, bevorzugt von ca. 10 bis ca. 13 Kohlenstoffatome, das Kation ist Natrium, Kalium, Ammonium, Mono-, Di- oder Triethanolammonium, Calcium oder Magnesium und Mischungen davon. Für milde Tensidsysteme ist Magnesium als Kation bevorzugt, für Standardwaschanwendungen dagegen Natrium. Gleiches gilt für Alkenylbenzolsulfonate.

Der Begriff anionische Tenside schließt auch Olefinsulfonate mit ein, die durch Sulfonierung von C₁₂-C₂₄-, vorzugsweise C₁₄-C₁₆-α-Olefinen mit Schwefeltrioxid und anschließende Neutralisation erhalten werden. Bedingt durch das Herstellverfahren, können diese Olefinsulfonate kleinere Mengen an Hydroxyalkansulfonaten und Alkandisulfonaten enthalten. Spezielle Mischungen von α-Olefinsulfonaten sind in US-A 3,332,880 beschrieben.

Weitere bevorzugte anionische Tenside sind Carboxylate, zum Beispiel Fettsäureseifen und vergleichbare Tenside. Die Seifen können gesättigt oder ungesättigt sein und können verschiedene Substituenten wie Hydroxylgruppen oder α-Sulfonatgruppen enthalten. Bevorzugt sind lineare gesättigte oder ungesättigte Kohlenwasserstoffreste als hydrophober Anteil mit ca. 6 bis ca. 30, bevorzugt ca. 10 bis ca. 18 Kohlenstoffatomen.

Als anionische Tenside kommen weiterhin in Frage: Salze von Acylaminocarbonsäuren; die durch Umsetzung von Fettsäurechloriden mit Natriumsarkosinat im alkalischen Medium entstehenden Acylsarkosinate; Fettsäure-Eiweiß-Kondensationsprodukte, die durch Umsetzung von Fettsäurechloriden mit Oligopeptiden erhalten werden; Salze von Alkylsulfamidocarbonsäuren; Salze von Alkyl- und Alkylarylethercarbonsäuren; C₈-C₂₄-Olefinsulfonate; sulfonierte Polycarboxylsäuren, hergestellt durch Sulfonierung der Pyrolyseprodukte von Erdalkalimetallcitraten, wie zum Beispiel beschrieben in GB-A 1 082 179; Alkylglycerinsulfate; Oleylglycerinsulfate; Alkylphenolethersulfate; primäre Paraffinsulfonate; Alkylphosphate; Alkyletherphosphate; Isethionate, wie Acylisethionate; N-Acyltauride; Alkylsuccinate; Sulfosuccinate; Monoester der Sulfosuccinate (besonders gesättigte und ungesättigte C₁₂-C₁₈-Monoester) und Diester der Sulfosuccinate (besonders gesättigte und ungesättigte C₁₂-C₁₈-Diester); Acylsarkosinate; Sulfate von Alkylpolysacchariden wie beispielsweise Sulfate von Alkylpolyglycosiden, verzweigte primäre Alkylsulfate und Alkylpolyethoxycarboxylate wie die der Formel RO(CH₂CH₂)ₖCH₂COO⁻M⁺, worin R C₈ bis C₂₂-Alkyl, k eine Zahl von 0 bis 10 und M ein Kation ist; Harzsäuren oder hydrierte Harzsäuren wie beispielsweise Rosin oder hydriertes Rosin oder Tallölharze und Tallölharzsäuren. Weitere Beispiele sind in "Surface Active Agents and Detergents" (Vol. I und II, Schwartz, Perry und Berch) beschrieben.

Ein Beispiel für ein kommerziell erhältliches anionisches Tensid ist Lutensit A-LBS der BASF Aktiengesellschaft.

Als nichtionische Tenside kommen beispielsweise folgende Verbindungen in Frage:

### - Polyethylen-, Polypropylen- und Polybutylenoxidkondensate von Alkylphenolen.

Diese Verbindungen umfassen die Kondensationsprodukte von Alkylphenolen mit einer C₆-C₂₀-Alkylgruppe, die entweder linear oder verzweigt sein kann, mit Alkenoxiden. Bevorzugt sind Verbindungen mit ca. 5 bis 25 mol Alkenoxid pro mol Alkylphenol.

### - Kondensationsprodukte von aliphatischen Alkoholen mit ca. 1 bis ca. 25 mol Ethylenoxid.

Die Alkylkette der aliphatischen Alkohole kann linear oder verzweigt, primär oder sekundär sein und enthält im Allgemeinen ca. 8 bis ca. 22 Kohlenstoffatome. Besonders bevorzugt sind die Kondensationsprodukte von C₁₀-C₂₀-Alkoholen mit ca. 2 bis ca. 18 mol Ethylenoxid pro mol Alkohol. Die Alkylkette kann gesättigt oder auch ungesättigt sein. Die Alkoholethoxilate können eine enge ("Narrow Range Ethoxylates") oder eine breite Homologenverteilung des Ethylenoxides ("Broad Range Ethoxylates") aufweisen.

Beispiele von kommerziell erhältlichen nichtionischen Tensiden dieses Typs sind beispielsweise die Lutensol^{®}-Marken der BASF Aktiengesellschaft, wie Lutensol AO 7, Lutensol TO 7 oder Lutensol XP 50 oder Edenor K8-18 sowie Edenor K12-18 der Firma Cognis, Deutschland.

### - Kondensationsprodukte von Ethylenoxid mit einer hydrophoben Basis, gebildet durch Kondensation von Propylenoxid mit Propylenglykol.

Der hydrophobe Teil dieser Verbindungen weist bevorzugt ein Molekulargewicht zwischen ca. 1.500 und ca. 1.800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind beispielsweise die Pluronic^{®}-Marken der BASF Aktiengesellschaft.

### - Kondensationsprodukte von Ethylenoxid mit einem Reaktionsprodukt von Propylenoxid und Ethylendiamin.

Die hydrophobe Einheit dieser Verbindungen besteht aus dem Reaktionsprodukt von Ethylendiamin mit überschüssigem Propylenoxid und weist im Allgemeinen ein Molekulargewicht von ca. 2.500 bis 3.000 auf. An diese hydrophobe Einheit wird Ethylenoxid bis zu einem Gehalt von ca. 40 bis ca. 80 Gew.-% Polyoxyethylen und einem Molekulargewicht von ca. 5.000 bis 11.000 addiert. Kommerziell erhältliche Beispiele dieser Verbindungsklasse sind beispielsweise die Tetronic^{®}-Marken der BASF Corp.

### - Semipolare nichtionische Tenside

Diese Kategorie von nichtionischen Verbindungen umfasst wasserlösliche Aminoxide, wasserlösliche Phosphinoxide und wasserlösliche Sulfoxide, jeweils mit einem Alkylrest von ca. 10 bis ca. 18 Kohlenstoffatomen. Semipolare nichtionische Tenside sind auch Aminoxide der Formel (IV) 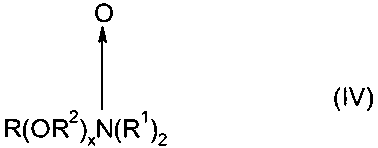 R ist hierbei eine Alkyl-, Hydroxyalkyl- oder Alkylphenolgruppe mit einer Kettenlänge von ca. 8 bis ca. 22 Kohlenstoffatomen. R² ist eine Alkylen- oder Hydroxyalkylengruppe mit ca. 2 bis 3 Kohlenstoffatomen oder Mischungen hiervon, jeder Rest R¹ ist eine Alkyl- oder Hydroxyalkylgruppe mit ca. 1 bis ca. 3 Kohlenstoffatomen oder eine Polyethylenoxidgruppe mit ca. 1 bis ca. 3 Ethylenoxideinheiten, und x bedeutet eine Zahl von 0 bis etwa 10. Die R¹-Gruppen können miteinander über ein Sauerstoff- oder Stickstoffatom verbunden sein und somit einen Ring bilden. Aminoxide dieser Art sind besonders C₁₀-C₁₈-Alkyldimethylaminoxide und C₈-C₁₂-A1 koxiethyl-Dihydroxyethylaminoxide.

### - Fettsäureamide

Fettsäureamide besitzen die Formel (V) 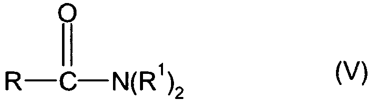 worin R eine Alkylgruppe mit ca. 7 bis ca. 21, bevorzugt ca. 9 bis ca. 17 Kohlenstoffatomen ist und R¹ unabhängig voneinander Wasserstoff, C₁-C₄-Alkyl, C₁-C₄-Hydroxyalkyl oder (C₂H₄0)ₓH bedeutet, wobei x von ca. 1 bis ca. 3 variiert. Bevorzugt sind C₈-C₂₀-Amide, - Monoethanolamide, -Diethanolamide und -Isopropanolamide.

Weitere geeignete nichtionische Tenside sind Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, alkoxylierte Triglycamide, Mischether oder Mischformale, Alkyloligoglycoside, Alkenyloligoglycoside, Fettsäure-N-alkylglucamide, Phosphinoxide, Dialkylsulfoxide und Proteinhydrolysate.

Typische Beispiele für amphotere bzw. zwitterionische Tenside sind Alkylbetaine, Alkylamidbetaine, Aminopropionate, Aminoglycinate oder amphotere Imidazolinium-Verbindungen der Formel (VI) 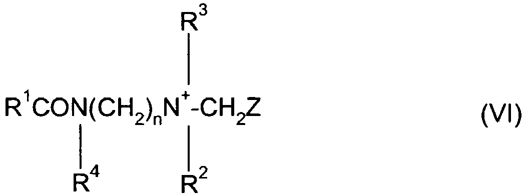 worin R¹ C₈-C₂₂-Alkyl- oder -Alkenyl, R² Wasserstoff oder CH₂CO₂M, R³ CH₂CH₂OH oder CH₂CH₂OCH₂CH₂CO₂M, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie ein Alkalimetall-, Erdalkalimetall-, Ammonium- oder Alkanolammoniumkation bedeutet.

Bevorzugte amphotere Tenside dieser Formel sind Monocarboxylate und Dicarboxylate. Beispiele hierfür sind Cocoamphocarboxypropionat, Cocoamidocarboxypropionsäure, Cocoamphocarboxyglycinat (auch als Cocoamphodiacetat bezeichnet) und Cocoamphoacetat.

Weitere bevorzugte amphotere Tenside sind Alkyldimethylbetaine und Alkyldipolyethoxybetaine mit einem Alkylrest mit ca. 8 bis ca. 22 Kohlenstoffatomen der linear oder verzweigt sein kann, bevorzugt mit 8 bis 18 Kohlestoffatomen und besonders bevorzugt mit 12 bis 18 Kohlenstoffatomen.

Geeignete kationische Tenside sind substituierte oder unsubstituierte geradkettige oder verzweigte quartäre Ammoniumsalze vom Typ R¹N(CH₃)₃⁺X⁻, R¹R²N(CH₃)₂⁺X⁻, R¹R²R³N(CH₃)⁺X⁻ oder R¹R²R³R⁴N⁺X⁻. Die Reste R¹, R², R³ und R⁴ sind unabhängig voneinander vorzugsweise unsubstituiertes Alkyl mit einer Kettenlänge von 8 bis 24 C-Atomen, insbesondere von 10 bis 18 C-Atomen, Hydroxyalkyl mit 1 bis 4 C-Atomen, Phenyl, C₂-C₁₈-Alkenyl, C₇-C₂₄-Aralkyl, (C₂H₄O)ₓH, wobei x eine ganze Zahl von 1 bis 3 bedeutet, ein oder mehrere Estergruppen enthaltende Alkylreste oder cyclische quartäre Amoniumsalze sein. X ist ein geeignetes, dem Fachmann bekanntes Anion.

### b) Tenside für maschinelle Geschirrreiniger

Bevorzugt werden schwach oder niedrig schäumende nichtionische Tenside in Anteilen von 0, 1 bis 20 Gew.-% (bevorzugt 0, 1 bis 10 Gew.-%, 0,25 bis 4 Gew.-%) eingesetzt. Dies sind beispielsweise Tenside aus der Gruppe der Fettalkoholethoxylate, wie sie z.B. kommerziell unter den Produktbezeichnungen Plurafac^{®} (BASF Aktiengesellschaft) oder Dehypon^{®} (Cognis) verfügbar sind. Weiterhin können Di- und Multiblockcopolymerisate aufgebaut aus Ethylenoxid und Propylenoxid eingesetzt werden, wie sie beispielsweise unter der Bezeichnung Pluronic^{®} (BASF Aktiengesellschaft) oder Tetronic^{®} (BASF Corporation) kommerziell erhältlich sind. Weiterhin können Umsetzungsprodukte aus Sorbitanestern mit Ethylenoxid und/oder Propylenoxid verwendet werden. Ebenfalls eignen sich Aminoxide oder Alkylglycoside. Eine Übersicht geeigneter nichtionischer Tenside geben beispielsweise die EP-A 0851 023 und die DE-A 198 19 187. Die Formulierung kann weiterhin anionische oder zwitterionische Tenside enthalten, bevorzugt in Abmischung mit nichtionischen Tensiden. Geeignete anionische und zwitterionischer Tenside sind ebenfalls in den Schriften EP-A 0851 023 und die DE-A 198 19 187 genannt.

### Lösungsmittel

Die erfindungsgemäßen Reinigerformulierungen können auch Lösungsmittel enthalten. Bevorzugt enthalten sie 0,1 bis 50 Gew.-%, mehr bevorzugt 1 bis 20 Gew.-%, mindestens eines Lösungsmittels, bezogen auf das Gesamtgewicht der Reinigerformulierung.

Als Lösungsmittel geeignet sind Alkohole wie Ethanol sowie Wasser. Bevorzugt wird als Lösungsmittel Wasser eingesetzt. Sofern Enzyme in den erfindungsgemäßen Reinigerformulierungen vorliegen, beträgt die Menge an eingesetztem Lösungsmittel, insbesondere an Wasser, vorzugsweise nicht mehr als 10 Gew.-% bezogen auf das Gesamtgewicht der Reinigerformulierung.

### Enzyme

Enzyme werden in Mengen von vorzugsweise 0,1 bis 10 Gew.-%, bezogen auf das Gesamtgewicht der Reinigerformulierung, verwendet.

Prinzipiell können alle dem Fachmann bekannten Enzyme verwendet werden, die gewöhnlicherweise in Reinigerformulierungen eingesetzt werden.

In einer bevorzugten Ausführungsform der vorliegenden Erfindung werden Enzyme eingesetzt, die den enzymatischen Abbau des Polymers ermöglichen, das das Bleichsystem schichtförmig umhüllt. Vorzugsweise handelt es sich bei diesen Enzymen, die den enzymatischen Abbau des Polymers ermöglichen, um lipolytische Enzyme. Als lipolytische Enzyme im Sinne der vorliegenden Erfindung werden Hydrolasen [EC 3.x.x.x] bezeichnet, beispielsweise Lipasen, Cutinasen, Esterasen, Polyesterasen, Peptidasen, Phospholipasen und Lysophospholipasen, vorzugsweise handelt es sich bei den Hydrolasen um Esterasen [EC 3.1.x.x] oder Peptidasen [EC 3.4.x.x]. Die vorstehenden EC-Nummern sind gemäß der EC-Klassifizierung (enzyme conmission number) aufgeführt und dem Fachmann geläufig. Konkrete Aminosäuresequenzen der Hydrolasen kann der Fachmann weiterhin geeigneten Genbanken entnehmen. Dies betrifft insbesondere die jeweiligen gi-Genbank-Zugangsnummern von NCBI, USA (http://www.ncbi.nlm.nih.gov/entrez/query.fcgi)

Die lipolytischen Enzyme stammen bevorzugt aus Mikroorganismen. Insbesondere stammen sie aus Bakterien, Pilzen oder Hefen. In einer bevorzugten Ausführungsform können die lipolytischen Enzyme stammen aus Absidia, insbesondere Absidia blakeslecna und Absidia corymbifera, Aspergillus, insbesondere Aspergillus niger und Aspergillus flavus, Achromobacter, insbesondere Achromobacter iophagus, Aureobasidium, insbesondere Aureobasidium pullulans, Bacillus, insbesondere Bacillus pumilus und Bacillus stearohermophilus, Brochotrix, insbesondere Brochotrix thermosophata, Candida, insbesondere Candida cylindracea (Candida rugosa), Candida paralypolitica und Candida antarctica, Chromobacter, insbesondere Chromobacter viscosum, Coprinus, insbesondere Coprinus cinerius, Fusarium, insbesondere Fusarium oxysporum und Fusarium solani, Geotricum insbesondere Geotricum penicillatum, Hansenula insbesondere Hansenula anomala, Humicola, insbesondere Humicola brevispora, Humicola brevis var. thermoidea und Humicola insolens, Hyphozyma, Lactobacillus, insbesondere Lactobacillus curvatus, Penicillium insbesondere Penicillium cyclopium, Penicillium crustosum und Penicillium expansum, Pseudomonas, insbesondere Pseudomonas aeruginosa, Pseudomonas cepacia, Pseudomonas fluorescens, Pseudomonas fragi, Pseudomonas mephitica, Pseudomonas alcaligenes, Pseudomonas plantari, Pseudomonas pseudoalcaligenes, Pseudonmonas putida, Pseudomonas mendocina oder Pseudomonas stutzeri, Rhizomucor, insbesondere Rhizomucor miehei, Rhizopus insbesondere Rhizopus japonius, Rhizopus micirosporus, Rhizopus delemar, Rhizopus niveus, Rhizopus arhizus und Rhizopus nodosus, Rhodotorula, insbesondere Rhodotorula glutinis, Sporobolomyces, insbesondere Sporobolomyces shibatanus, Thermomyces, insbesondere Thermomyces lanuginosus (früher Humicola lanuginosa), Thiarosporella, insbesondere Thiarosporella phaseolina und/oder Trichoderma insbesondere Trichoderma harzanium, Trichoderma reesei. Weiter können die lipolytischen Enzyme auch pflanzlichen oder tierischen Ursprungs sein. Geeignete lipolytische Enzyme werden beispielsweise in WO 02/095127 offenbart und sind unter Bezugnahme in die vorliegende Erfindung mit aufgenommen.

In einer ganz besonders bevorzugten Ausführungsform sind die lipolytischen Enzyme gemäß dieser Erfindung Lipasen [3.1.1.3] aus dem Stamm von Candida cylindracea, einem Stamm von Candida antarctica insbesondere die Lipase B aus Candida antarctica (WO 88/02775), aus einem Stamm von Pseudomonas cepacia, einem Stamm von Hyphozyma, einem Stamm von Aspergillus niger und/oder einem Stamm von Mucor mihei.; oder die Enzyme sind ausgewählt aus der Enzymklasse der Subtilisine [EC 3.4.21.62], wie zB die kommerziell erhältlichen Subtilisin-Formulierungen "Savinase" oder "Alcalase" der Firma Novozymes. Sanivasen sind kommerziell beispielsweise als Handelsprodukt Savinase 16L, Type X von Novozymes A15, Bugsvaerd, Dänemark erhältlich.

Lipasen aus der Fraktion B von Candida antarctica, die im erfindungsgemäßen Verfahren verwendet werden können, haben bevorzugt eine Aminosäuresequenz wie in einer der nachfolgenden Genbank-Zugangsnummer hinterlegt [gi-Genbank-Zugangsnummern von NCBI, USA (http://www.ncbi.nlm.nih.gov/entrez/query.fcgi)]: gi:1085991, gi:1170790, gi: 1311320, gi:576303, gi:567302, gi:576301, gi:576300, gi:576299 oder gi:515792.

Savinasen, die im erfindungsgemäßen Verfahren verwendet werden können, haben bevorzugt eine Aminosäuresequenz wie in einer der nachfolgenden Genbank-Zugangsnummer hinterlegt: gi:267048, gi:50513761, gi:50513760, gi:1827586, gi:85362336, gi:85362332, gi:85362328 oder gi:85362326.

Alcalasen, die im erfindungsgemäßen Verfahren verwendet werden können, haben bevorzugt eine Aminosäuresequenz wie in einer der nachfolgenden Genbank-Zugangsnummer hinterlegt: gi:135016 oder gi:135015.

In einer anderen bevorzugten Ausführungsform ist das lipolytische Enzym eine Esterase, die aus einem Stamm von Rhodosporidium, insbesondere Rhodosporidium toruloides oder einem Stamm von Pseudomonas insbesondere Pseudomonas aerigunosa, Pseudomonas pseudoalcaligenes, Pseudomonas fluorescens, Pseudomonas putidu und Pseudomonas maltophilia.

Bevorzugt stammen die Proteasen aus Bakterien der Gattung Bacillus, besonders bevorzugt eigenen sich Proteasen der Organismen Bacillus alcalophilus und Bacillus licheniformis.

Geeignete Mikroorganismen zur Herstellung der erfindungsgemäß geeigneten Enzyme, wie beispielsweise Candida antarctica, können nach den üblichen Methoden der Mikrobiologie isoliert werden, z.B. durch Anzucht auf üblichen Nährmedien und Prüfung auf Lipase-Aktivität. Die Isolierung und Reinigung der Enzyme erfolgt ebenfalls nach den üblichen Methoden (vgl. z.B. WO 88/02775).

In einer weiteren bevorzugten Ausführungsform werden Polyesterasen als Enzyme eingesetzt, die einen Abbau des Polymers ermöglichen, das das Bleichsystem schichtförmig umhüllt. Geeignete Polyesterasen sind beispielsweise in WO 01/14629 beschrieben und unter Bezugnahme in die vorliegende Erfindung mit aufgenommen.

Bevorzugte Polyesterasen sind Polyesterasen aus Absidia species, Acremonium species, Agaricus species, Anaeromyces species, Aspergillus species, Aeurobasidium species, Cephalosporum species, Chaetomium species, Coprinus species, Dactyllum species, Fusarium species, Gliocladium species, Humicola species, einschließlich H. insolens und H. lanuginose, Mucor species, Neurospora species, Neocallimastix species, Orpinomyces species, Penicillium species, Phanerochaete species, Phlebia species, Piromyces species, Pseudomonas species, Rhizopus species, Schizophyllum species, Trametes species, Trichoderma species, Zygorhynchus species, Bacillus species, Cellulomonas species, Clostridium species, Myceliopphthora species, Thermomonospora species, Streptomyces species, Fibrobacter species, Candida species, Pichia minuta, Rhodotorula glutinis, R. mucilaginosa, Sporobolomyces holsaticus oder Thermomyces species.

In einer weiteren bevorzugten Ausführung für den enzymatischen Abbau der Polymere, die das Bleichsystem schichtförmig umhüllen, wird die Lipase aus Candida antarctica Komponente B, die Lipase aus Aspergillus niger oder die Lipase Lipozyme 20.000 L oder eine Mischung davon verwendet. Diese Enzyme können auch jeweils oder in Mischung mit weiteren Enzymen eingesetzt werden.

Das Verhältnis, in dem Enzyme in Kombination eingesetzt werden, ist durch deren Aktivität gegenüber dem Polymer oder seinen Abbauprodukten festgelegt. Die Enzyme können in einem Aktivitätsverhältnis 5 : 95 bis 95 : 5 eingesetzt werden, bevorzugt beträgt das Verhältnis 20 : 80 bis 80 : 20 und besonders bevorzugt 40 : 60 oder 60 : 40. Die Bestimmung der Aktivität erfolgt beispielsweise über Freisetzung saurer Gruppen während des enzymatischen Polymerabbaus mittels Titration. Es können weitere lipolytische und/oder proteolytische Enzyme eingesetzt werden.

Weiterhin können Metallionen, wie beispielsweise Natrium- oder Calciumionen, zugesetzt werden. Anionische oder nichtionische Tenside wie beispielsweise sekundäre Alkoholethoxylate können ebenfalls zugesetzt werden.

Die erfindungsgemäß verwendbare Lipase (B) aus dem Stamm Candida antarctica Komponente B ist beschrieben in WO 88/02775. Die Lipase Lipozyme 20.000 L ist ein Handelsprodukt der Fa. Novozymes, Dänemark. Die Lipase aus dem Stamm Aspergillus niger ist käuflich zu erhalten beispielsweise bei der Fa. Fluka, Buchs, Liechtenstein.

Neben den vorgenannten Enzymen, die einen enzymatischen Abbau des Polymers ermöglichen, das das Bleichsystem schichtförmig umhüllt, können die erfindungsgemäßen Reinigerformulierungen auch weitere Enzyme enthalten, die gewöhnlich in Reinigerformulierungen enthalten und dem Fachmann bekannt sind. Für Reinigerformulierungen, die als Haushaltstextilwaschmittel geeignet sind, sind dies insbesondere Proteasen, Amylasen und Cellulasen. Vorzugsweise geeignet sind hierfür Cellulasen. Die hierbei verwendete Cellulase kann aus Bakterien oder Pilzen gewonnen sein und soll einen optimalen pH-Bereich zwischen 5 und 9,5 aufweisen. Geeignete Cellulasen sind in US 4.435.307 offenbart. Es handelt sich hierbei um Cellulase, die von einem Stamm von Humicola insolens produziert wird, insbesondere vom Stamm Humicola DSM 1800 oder einem anderen Cellulase-212-produzierenden Pilz, der zur Gattung Aeromonas gehört sowie Cellulase, die aus dem Hepatopankreas bestimmter mariner Mollusken extrahiert wurde. Geeignete Cellulasen sind ebenfalls in GB-A 2 075 028, GB-A 2 085 275 und DE-OS 2 247 832 offenbart.

Bevorzugte Cellulasen sind in WO-91/17243 beschrieben. Die erfindungsgemäßen Reinigungsmittelzusammensetzungen enthalten solche weiteren Enzyme in Mengen bis etwa 50 mg, bevorzugt von etwa 0,01 mg bis etwa 10 mg pro Gramm der Reinigungsmittelzusammensetzung. Bezogen auf das Gewicht der Waschmittelzusammensetzungen beträgt der Anteil der Enzyme - sofern sie vorhanden sind - mindestens 0,001 Gew.-%, bevorzugt etwa 0,001 Gew.-% bis etwa 5 Gew.-%, insbesondere von etwa 0,001 Gew.-% bis etwa 1 Gew.-%, speziell von etwa 0,01 Gew.-% bis etwa 1 Gew.-%.
Im Fall von maschinellen Geschirrreinigern eignen sich die folgenden Enzyme, wobei bei einer solchen Reinigerformulierung zwischen 0 und 5 Gew.-% Enzyme bezogen auf die gesamte Zubereitung zugesetzt werden können, um die Leistung der Reinigungsmittel zu steigern oder unter milderen Bedingungen die Reinigungsleistung in gleicher Qualität zu gewährleisten. Zu den am häufigsten verwendeten Enzymen gehören Lipasen, Amylasen, Cellulasen und Proteasen. Weiterhin können auch Esterasen, Pectinasen, Lactasen und Peroxidasen eingesetzt werden. Bevorzugte Proteasen sind z.B. BLAP^{e}l40 der Fa. Biozym, Optimase^{®} M-440 und Opticlean^{®} M-250 der Fa. Solvay Enzymes; Maxacal^{®} CX und Maxapem^{®} oder Esperase^{®} der Fa. Gist Brocades oder Savinase^{®} der Fa. Novo oder Purafect OxP der Fa. Genencor. Besonders geeignete Cellulasen und Lipasen sind Celluzym^{®} 0,7T und Lipolase^{®} 30T der Fa. Novozymes Besondere Verwendung als Amylasen finden Duramyl^{®} und Termamyl^{®} 60 T, und Termamyl^{®} 90 T der Fa. Novo, Amylase-LT^{®} der Fa. Solvay Enzymes, Maxamyl^{®} P5000 der Fa. Gist Brocades oder Purafect^{®} OxAm der Fa. Genencor. Auch andere Enzyme können verwendet werden.

### Weitere Zusatzstoffe

Die erfindungsgemäßen Reinigerformulierungen können weiterhin 0,1 bis 90 Gew.-%, bezogen auf die Gesamtmenge der Reinigerformulierungen, an mindestens einem weiteren Zusatzstoff enthalten.

Als weitere Zusatzstoffe sind insbesondere geeignet: Schaumverstärker, Schaumbremsen, Anlauf- und/oder Korrosionsschutzmittel, Suspensionsmittel, Farbstoffe, Füllmittel, optische Aufheller, Desinfektionsmittel, Alkalien, hydrotrope Verbindungen, Antioxidantien, Enzymstabilisatoren, Parfüme, Lösungsmittel, Lösungsvermittler, Wiederablagerungsverhinderer, Dispergiermittel, Farbübertragungsinhibitoren, z. B. Polyamin-N-oxide wie etwa Poly-(4-vinylpyridin-N-oxid), Polyvinylpyrrolidon, Poly-N-vinyl-N-methylacetamid und Copolymere von N-Vinylimidazol und N-Vinylpyrrolidon, Verarbeitungshilfsmittel, Netzmittel, Weichmacher und Antistatikhilfsmittel. Wie vorstehend bereits beschrieben können die erfindungsgemäßen Reinigerformulierungen weiterhin auch Bleichmittel, Bleichaktivator oder Bleichkatalysatoren enthalten, die nicht mit mindestens einem Polymer schichtförmig umhüllt sind und wobei das Polymer Urethan- und Harnstoffgruppen aufweist (also nicht-umhüllte Bleichsystem-Komponenten).

Im Fall von Reinigerformulierungen, die als Haushaltstextilwaschmittel geeignet sind, handelt es sich vorzugsweise um anorganische und/oder organische Gerüststoffe, die den Härtegrad des Wassers vermindern.

Diese Gerüststoffe können mit Gewichtsanteilen von etwa 5 % bis etwa 80 % in den Wasch- und Reinigungsmittelzusammensetzungen enthalten sein. Anorganische Gerüststoffe umfassen beispielsweise Alkali-, Ammonium- und Alkanolammoniumsalze von Polyphosphaten wie beispielsweise Tripolyphosphate, Pyrophosphate und glasartige polymere Metaphosphate, Phosphonate, Silikate, Carbonate einschließlich Bicarbonate und Sesquicarbonate, Sulfate und Aluminosilikate.

Beispiele für Silikatgerüststoffe sind die Alkalimetallsilikate, insbesondere diejenigen mit einem SiO₂:Na₂O-Verhältnis zwischen 1,6 : 1 und 3,2 : 1 sowie Schichtsilikate, beispielsweise die in der US 4,664,839 beschriebenen Natriumschichtsilikate, erhältlich von Clariant GmbH unter der Marke SKS^{®}. SKS-6^{®} ist ein besonders bevorzugter Schichtsilikatgerüststoff.

Aluminosilikatgerüststoffe sind für die vorliegende Erfindung besonders bevorzugt. Es handelt sich dabei insbesondere um Zeolithe mit der Formel Na_{z}[(AlO₂)_{z}(SiO₂]_{Y} xH₂O, worin z und y ganze Zahlen von mindestens 6 bedeuten, das Verhältnis von z zu y von etwa 1,0 bis 0,5 liegt und x eine ganze Zahl von 15 bis 264 bedeutet.

Geeignete Ionentauseher auf Aluminosilikatbasis sind im Handel erhältlich. Diese Aluminosilikate können von kristalliner oder amorpher Struktur sein und können natürlich vorkommend oder auch synthetisch hergestellt sein. Verfahren für die Herstellung von lonentauschern auf Aluminosilikatbasis werden beispielsweise beschrieben in US 3,985,669 und US 4,605,509. Bevorzugte Ionentauseher auf der Basis synthetischer kristalliner Aluminosilikate sind erhältlich unter der Bezeichnung Zeolith A, Zeolith P (B) (einschließlich der in EP-A 0 384 070 offenbarten) und Zeolith X. Bevorzugt sind Aluminosilikate mit einem Partikeldurchmesser zwischen 0,1 und 10 µm.

Geeignete organische Gerüststoffe umfassen Polycarboxylverbindungen wie beispielsweise Etherpolycarboxylate und Oxydisuccinate, wie beispielsweise in US 3,128,287 und US 3,635,830 beschrieben. Ebenfalls sind die aus US 4,663,071 bekannten TMS/TDS-Gerüststoffe geeignet.

Andere geeignete Gerüststoffe umfassen die Etherhydroxypolycarboxylate, Copolymere von Maleinsäureanhydrid mit Ethylen oder Vinylmethylether, 1,3,5-Trihydroxybenzol-2,4,6-trisulfonsäure und Carboxymethyloxybernsteinsäure, die Alkali-, Ammonium- und substituierten Ammoniumsalze von Polyessigsäuren wie z. B. Ethylendiamintetraessigsäure und Nitrilotriessigsäure sowie Polycarbonsäuren wie Mellithsäure, Bernsteinsäure, Oxydibernsteinsäure, Polymaleinsäure, Benzol-1,3,5-tricarbonsäure, Carboxymethyloxybernsteinsäure sowie deren lösliche Salze.

Gerüststoffe auf Citratbasis, z. B. Zitronensäure und ihre löslichen Salze, insbesondere das Natriumsalz, sind bevorzugte Polycarbonsäuregerüststoffe, die auch in granulierten Formulierungen, insbesondere zusammen mit Zeolithen und/oder Schichtsilikaten verwendet werden können.

Weitere geeignete Gerüststoffe sind die 3,3-Dicarboxy-4-oxa-1,6-hexandioate und die verwandten Verbindungen, die in US 4,566,984 offenbart sind.

Wenn Gerüststoffe auf Phosphorbasis verwendet werden können und insbesondere, wenn Seifenstücke für die Wäsche von Hand formuliert werden sollen, können verschiedene Alkalimetallphosphate wie etwa Natriumtripolyphosphat, Natriumpyrophosphat und Natriumorthophosphat verwendet werden. Ebenfalls können Phosphonatgerüststoffe wie Ethan-1-hydroxy-1,1-diphosphonat und andere bekannte Phosphonate, wie sie beispielsweise in US 3,159,581, US 3,213,030, US 3,422,021, US 3,400,148 und US 3,422,137 offenbart sind, verwendet werden.

Weitere Zusatzstoffe, die für Reinigerformulierungen geeignet sind, die als maschinelle Geschirrreiniger verwendet werden, sind vorzugsweise wie folgt definiert.

### Builder:

Es können wasserlösliche und wasserunlösliche Builder eingesetzt werden deren Hauptaufgabe im Binden von Calcium und Magnesium besteht. Übliche Builder, die zwischen 10 und 90 Gew.-% bezogen auf die gesamte Zubereitung zugegen sein können, sind nachfolgend aufgeführt.

Phosphate wie beispielsweise Alkaliphosphate und polymere Alkaliphosphate, die in Form ihrer alkalischen, neutralen oder sauren Natrium- oder Kaliumsalze vorliegen können. Beispiele hierfür sind Trinatriumphosphat, Tetranatriumdiphosphat, Dinatriumdihydrogenphosphat, Pentanatriumtripolyphosphat, sogenanntes Natriumhexametaphosphat, oligomeres Trinatriumphosphat mit Oligomerisierungsgraden von 5 bis 1000, insbesondere von 5 bis 50, sowie die entsprechenden Kaliumsalze bzw. Gemische aus Natriumhexametaphosphat und die entsprechenden Kaliumsalze bzw. Gemische aus Natrium- und Kaliumsalzen. Diese Phosphate werden vorzugsweise im Bereich von 25 Gew.-% bis 65 Gew.-% bezogen auf die gesamte Rezeptur und berechnet als wasserfreie Aktivsubstanz eingesetzt.

Niedermolekulare Carbonsäuren und ihre Salze, wie z.B. Alkalicitrate (wie z.B. wasserfreies Trinatriumcitrat oder Trinatriumcitratdihydrat), Alkalisuccinate, Alkalimalonate, Fettsäuresulfonate, Oxydisuccinate, Alkyl- oder Alkenyldisuccinate, Gluconsäuren, Oxadiacetate, Carboxymethyloxysuccinate, Tartratmonosuccinat, Tartratdisuccinat, Tartratmonoacetat, Tartratdiacetat, α-Hydroxypropionsäure, oxidierte Stärken, oxidierte Polysaccharide; homo-und copolymere Polycarbonsäuren und ihre Salze, wie z.B. Polyacrylsäure, Polymethacrylsäure, Copolymer Maleinsäure/Acrylsäure, Copolymer Maleinsäure/Acrylsäure/Vinylacetat; Pfropfpolymerisate von monoethylenisch ungesättigten Mono- und/oder Dicarbonsäuren auf Monosaccharide, Oligosaccharide, Polysaccharide oder Polyasparaginsäure; Aminopolycarboxylate und Polyasparaginsäure; Carbonate, wie beispielsweise Natriumcarbonat und Natriumbicarbonat.

Komplexbildner und Phosphonate und deren Salze, wie z.B. Nitrilotriessigsäure, Ethylendiamintetraessigsäure, Diethylentriaminpentaessigsäure, Hydroxyethylethylendiamintriessigsäure, Methylglycindiessigsäure, 2-Phosphono-1,2,4-butantricarbonsäure, Aminotri-(methylenphosphonsäure), 1-Hydroxyethylen(1,1-diphosphonsäure), Ethylendiamin-tetramethylenphosphonsäure, Hexamethylendiamintetramethylenphosphonsäure und Diethylentriaminpentamethylenphosphonsäure.

Silikate wie beispielsweise Natriumdisilikat und Natriummetasilikat. Zu wasserunlöslichen Buildern zählen die Zeolithe und kristallinen Schichtsilikate, wobei letztere insbesondere der allgemeinen Formel NaMSiₓO₂ₓ₊₁ * y H₂O entsprechen, wobei M Natrium oder Wasserstoff darstellt, x eine Zahl von 1,9 bis 22, vorzugsweise 1,9 bis 4, ist und y für eine Zahl von 0 bis 33 steht. Bekannte Beispiele hierfür sind insbesondere α-Na₂Si₂O₅, β-Na₂Si₂O₅, δ-Na₂Si₂O₅. Ebenso zählen hierzu Mischungen der vorgenannten Buildersubstanzen. Bevorzugt werden Trinatriumcitrat und/oder Pentanatriumtripolyphosphat und/oder Natriumcarbonat und/oder Natriumbicarbonat und/oder Gluconate und/oder silikatische Builder aus der Klasse der Disilikate und/oder Metasilikate eingesetzt.

Ein Beispiel für einen kommerziell erhältlichen Builder ist Sokalan HP 25 der BASF Aktiengesellschaft, Ludwigshafen, Deutschland.

### Alkaliträger:

Als weitere Bestandteile können Alkaliträger zugegen sein. Als Alkaliträger gelten Alkalimetallhydroxide, Alkalimetallcarbonate, Alkalimetallhydrogencarbonate, Alkalimetallsesquicarbonate, Alkalisilikate, Alkalimetasilikate und Mischungen der vorgenannten Stoffe, wobei bevorzugt die Alkalicarbonate, insbesondere Natriumcarbonat, Natriumhydrogencarbonat oder Natriumsesquicarbonat eingesetzt werden. Bevorzugte Kombinationen aus Builder und Alkaliträger sind Mischungen aus Tripolyphosphat und Natriumcarbonat bzw. Tripolyphosphat, Natriumcarbonat und Natriumdisilikat.

### Korrosionsinhibitoren:

Insbesondere können Silberschutzmittel aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden bevorzugt sauerstoff- und stickstoffhaltige organische redoxaktive Verbindungen, wie zwei- und dreiwertige Phenole, z.B. Hydrochinon, Brenzkatechin, Hydroxyhydrochinon, Gallussäure, Phloroglucin, Pyrogallol bzw. Derivate dieser Verbindungsklassen eingesetzt. Auch salz- und komplexartige anorganische Verbindungen, wie Salze der Metalle Mn, Ti, Zr Hf, V, Co und Ce finden häufig Verwendung. Bevorzugt sind hierbei die Übergangsmetallsalze, die ausgewählt sind aus der Gruppe der Mangan- und Cobaltsalze und deren Komplexen, besonders bevorzugt der Cobalt(ammin)-Komplexe, der Cobalt(acetat)-Komplexe, der Cobalt-(Carbonyl)-Komplexe, der Chloride des Cobalts und des Mangans und des Mangansulfats. Ebenfalls können Zinkverbindungen oder Wismutverbindungen zur Verhinderung der Korrosion am Spülgut eingesetzt werden.

### Weitere Zusätze:

Paraffinöle und Silikonöle können optional als Entschäumer und zum Schutz von Kunststoff- und Metalloberflächen eingesetzt werden. Entschäumer werden generell in Anteilen von 0,001 % bis 5% dosiert.

Gewerbliche Reinigertypen enthalten meist ein Buildersystem auf Basis von Pentanatriumtriphosphat, und/oder Natriumcitrat und/oder Komplexbildnern wie z.B., Nitrilotriacetat. Häufig wird im Gegensatz zu Haushaltsreinigern mit Natronlauge oder Kalilauge als Alkaliträger gearbeitet

Bevorzugte Reinigerformulierungen enthalten zu
i) 0,1 bis 30 Gew.-% mindestens ein Bleichsystem,
ii) 0,1 bis 99,9 Gew.-% mindestens ein Tensid,
iii) 0 bis 50 Gew.-% mindestens ein Lösungsmittel,
iv) 0 bis 10 Gew.-% mindestens ein Enzym und
v) 0 bis 90 Gew.-% mindestens einen weiteren Zusatzstoff,
wobei das Verhältnis der Komponenten i) bis v) so gewählt ist, dass die Summe 100 Gew.-% ergibt

Ein weiterer Gegenstand der vorliegenden Erfindung ist die Verwendung eines der vorstehend beschriebenen Bleichsysteme oder einer der vorbeschriebenen Reinigerformulierungen enthaltend mindestens ein solches Bleichsystem zum Reinigen oder Waschen von Haushaltstextilien oder von Geschirr im Haushaltsbereich oder im gewerblichen Bereich, als Fleckensalz, als Desinfektionsmittel und in der Zellstoffbleiche, der Holzstoffbleiche, der Baumwollfaserbleiche und der Haarbleiche.

Besonders bevorzugt wird das erfindungsgemäße Bleichsystem als Bestandteil einer Reinigerformulierung (beziehungsweise die erfindungsgemäßen Reinigerformulierungen selber) in Form eines gelförmigen oder flüssigen Textilwaschmittels oder Reinigers verwendet.

Die vorliegende Erfindung soll anhand der nachfolgenden Beispiele näher erläutert werden.

### Herstellung des Polymers (in Dispersionsform)

### Beispiel Ia

In einem Rührkolben werden vorgelegt:

800 g (0,40 mol) eines Polyesterols aus Isophthalsäure, Adipinsäure und 1,6-Hexandiol der OH-Zahl 56 mg/g, 80,4 g (0,60 mol) DMPA und 36,0 g (0,40 mol) Butandiol-1,4.

Dazu gibt man bei 105°C 400 g (1,80 mol) IPDI und 160 g Aceton. Nach vier Stunden Rühren bei 105°C wird mit 1600 g Aceton verdünnt. Der Gehalt Rest -NCO der Lösung wird zu 1,11% bestimmt (berechnet: 1,08%).

Die Lösung wird auf 45°C gekühlt und mit 68,0 g (0,40 mol) IPDA versetzt. Nach 90 Minuten wird mit 50,0 g (0,73 mol) 25%igen wässrigem Ammoniak neutralisiert, mit 3000 g Wasser dispergiert und das Aceton i.V. abgezogen.

Man erhält eine fast transparente Dispersion mit einem Feststoffgehalt von 30 Gew.-%.

Ein Gießfilm dieser Dispersion weist eine Reißspannung von 29 MPa bei einer Reißdehnung von 415% auf (Zugversuch nach DIN 53504).

### Herstellung eines Bleichsystems, das mit Polymer schichtförmig umhüllt ist

### Beispiel Ib

In einem Wirbelschichttrockner werden 2615g 6-(Phthalimido)peroxyhexansäure [Eureco^{®} der Firma Solvay; PAP; CAS Nr. 128275-31-0] mit 7,5 kg einer 25%igen Dispersion gemäß Beispiel Ia unter den aufgeführten Bedingungen beschichtet:
Zulufttemperatur 45-47 °C; Ablufttemperatur 39-41 °C; Luftdruck 1004 mbar; rel. Feuchte 42 %; Luftmenge 415 m³ /h; reine Sprühzeit 6 h.
Rückwaage: 4360 g (1745g Auflage, 40%iger); Verbackungen 140 g.

Zum Abschluss wird das Granulat für 10 min auf 50°C getempert. Es wird ein frei fließendes weißes Granulat erhalten, das sich in untenstehenden flüssigen Waschmittelkonzentraten ohne Verfärbung des Peroxidindikators (Indigo-5,5',7,7'trisulfonsäure, Trikaliumsalz) bei Raumtemperatur über 20 Wochen lagern lässt.

### Flüssige Waschmittelkonzentrate

Folgende verschiedene Waschmittelkonzentrate werden getestet (Angaben in Gew.-%):

| | **W1** | **W2** | **W3** | **W4** |
|---|---|---|---|---|
| Lutensit A-LBS (98%) | 28 | 22 | 22 | 20 |
| Lutensol AO 7, TO 7 | 20 | 27 | 27 | 10,8 |
| Lutensol XP 50 | | | | 16,2 |
| Fettsäure Edenor K8-18 | 20 | | | |
| Fettsäure Edenor K12-18 | | 15 | 15 | 15 |
| Sokalan HP 25 (45%) | | | | 4 |
| Monopropylenglykol | 17 | 10 | 20 | 10 |
| Emulan HE 50 | | 10 | | 10 |
| Ethanol | | 4 | 4 | 2,7 |
| Monoethanolamin | 11,63 | 9,21 | 9,21 | 8,76 |
| Wasser | 3,37 | 2,79 | 2,79 | 2,54 |

### Herstellung der flüssigen Waschmittelkonzentrate

Nichtionisches Tensid (Lutensol AO7; TO7), Monopropylenglykol (Weichmacher) und gegebenenfalls Emulan HE50 (Netzmittel) vorlegen, Lutensit A-LBS zuwiegen, mit Monoethanolamin neutralisieren (gegebenenfalls kann Teil des Monoethanolamins durch KOH ersetzt werden), Fettsäure zuwiegen und mit Restmenge Monoethanolamin neutralisieren, Wasser, Ethanol, Sokalan HP25 zugeben

### Reinigerformulierungen

Standardprüfung im Launderometer bei 50°C an Tee-, Rotwein-, Gras- und Curry- Gewebe. Zusätzlich wird die Entfernung von Triolein- und Olivenöl- Flecken von Baumwollgewebe untersucht. Dazu wird Triolein und Olivenöl mit 0,1% Sudanrot 7 B (= Solvent Red 19) eingefärbt. Dieser Farbstoff wird zuvor unter leichtem Erwärmen gelöst und homogenisiert. Die Präparation erfolgt durch Auftropfen mit einer Pipette, wobei das Prüfgewebe aufgespannt wird. 0,1 g der Lösung werden aufgetropft und über Nacht auf dem Gewebe ausgespreitet. Alle Prüfmuster werden doppelt bestimmt.

Dazu wird den flüssigen Waschmittelkonzentraten (W1 - W4) 15 Gew.-% des Beispiels Ib und 0,1 Gew.-% Savinase (Handelsprodukt Savinase 16L, Type Ex von Novozymes A/S, Bagsvaerd, Dänemark) zugemischt und trocken homogenisiert.

Die Auswertung der Bleichversuche im Launderometer erfolgt über Remissionsmessung und Berechnung der Bleichwirkung an den verwendeten bleichbaren Anschmutzungen (Tee, Rotwein, Gras, Curry, Triolein und Olivenöl). Die Standardabweichung der Remmision R ist <1%.

### Waschbedingungen

| | |
|---|---|
| Waschgerät | Launder-O-Meter |
| Wasserhärte | 3 mmol Ca⁺⁺+Mg⁺⁺/1 = 16,8 °dH |
| Verhältnis Ca : Mg : NaHCO₃ | 4:1:8 mol |
| Waschtemperatur 1 | 25°C |
| Waschtemperatur 2 | 50°C |
| Waschzeit | 30 min |
| Waschzyklen | je 1 |
| Waschmitteldosierung | 4,5 g/l |
| Flottenverhältnis | 1:20 |
| Gesamtflotte | 250 ml |
| Gewebe | 2,5 g BW/Tee |
| | 2,5 g BW/Rotwein (EMPA 114) |
| | 2,5 g BW/Gras (CFT-AS 4, Chlorophyll-Pflanzenöl) |

### Gewebevorbereitung

Für das Bleichgewebe EMPA 114 muss im Auswerteprogramm (CGTec) eine Gewebecharge angelegt sein. Nicht kommerziell erhältliche Anschmutzungen wie Tee, Chlorophyll wurden einzeln vorgemessen.

### Härte

Ansetzen von Stammhärte 1 (Ca⁺⁺ + Mg⁺⁺) und Stammhärte 2 (NaHCO₃) beide je 0,8 mol/l. Härtewasser wird vor Gebrauch überprüft. Anwendung findet die Bestimmung der Wasserhärte mit Titriplex-Lösung.

| | |
|---|---|
| Stammhärte 1 | 94,09 g Calciumchlorid * 2 H₂O und |
| | 32,53 g Magnesiumchlorid * 6 H₂O auf 1 Liter im Messkolben mit VE-Wasser auffüllen |
| Stammhärte 2 | 67,2 g Natriumhydrogencarbonat auf 1 Liter im Messkolben mit VE-Wasser auffüllen |
| Härtewasser 1: | 39,06 g Stammhärte 1 auf 1 l mit VE-Wasser auffüllen |
| Härtewasser 2: | 62,50 g Stammhärte 2 auf 1 l mit VE-Wasser auffüllen |

### Spülwasser ist Trinkwasser

### Waschen

| Wäsche 50°C: | |
|---|---|
| Anfangstemperatur | 25°C, 10 min Aufheizzeit (2,5 °C/min) |
| Waschtemperatur | 20 min bei 50°C |
| Endtemperatur | 25°C, ca. 6 min Abkühlzeit |

Die nachfolgende Vorschrift beschreibt die Durchführung des Bleichversuchs. Der Bleichversuch besteht aus 2 Wäschen mit den vorstehend beschriebenen flüssigen Waschmittelformulierungen, denen jeweils die Bleichsubstanz und das Enzym separat zugegeben wird. Die Bleichwirkung wird an verschiedenen Bleich-Testgeweben bei verschiedenen Temperaturen untersucht.

### Reproduzierbarkeit unter Wiederholbedingungen

Um die Wiederholbarkeit der Methode zu überprüfen, wurde der Bleichversuch 6 mal durchgeführt. Additiv bedeutet Beispiel lb und Enzym.

| Formulierung **[W1]** *-ohne lb-* | Vertrauensbereich Bleichwirkung in % |
|---|---|
| Gewebe Baumwolle/Tee | 28,8 ± 7,7 |
| Gewebe Baumwolle/Rotwein | 54,6 ± 3,5 |
| Gewebe Baumwolle/Gras | 39,8 ± 5,7 |
| Gewebe Baumwolle/Curry | 63,7 ± 4,5 |
| Gewebe Baumwolle/Olivenöl | 39,1 ± 3,5 |

| Formulierung **[W1]** *-mit lb-* | Vertrauensbereich Bleichwirkung in % |
|---|---|
| Gewebe Baumwolle/Tee | 53,2 ± 6,7 |
| Gewebe Baumwolle/Rotwein | 67,7 ± 5,5 |
| Gewebe Baumwolle/Gras | 42,8 ± 4,7 |
| Gewebe Baumwolle/Curry | 70,7 ± 3,5 |
| Gewebe Baumwolle/Olivenöl | 48,1 ± 3,5 |

## Patentansprüche

1. Bleichsystem enthaltend mindestens eine Komponente ausgewählt aus Bleichmittel, Bleichaktivator oder Bleichkatalysator, **dadurch gekennzeichnet, dass** das Bleichsystem mit mindestens einem Polymer schichtförmig umhüllt ist und das Polymer Urethan- und Harnstoffgruppen aufweist.

2. Bleichsystem gemäß Anspruch 1, **dadurch gekennzeichnet, dass** das mit mindestens einem Polymer schichtförmig umhüllte Bleichsystem partikelförmig ist.

3. Bleichsystem gemäß Anspruch 1 oder 2, **dadurch gekennzeichnet, dass** der mittlere Partikeldurchmesser 0,01 bis 5 mm beträgt.

4. Bleichsystem gemäß einem der Ansprüche 1 bis 3, **dadurch gekennzeichnet, dass** das Polymer eine Schichtdicke von 10 bis 2000 µm aufweist.

5. Bleichsystem gemäß einem der Ansprüche 1 bis 4, **dadurch gekennzeichnet, dass** das Bleichsystem mindestens ein Bleichmittel, aber keinen Bleichaktivator und keinen Bleichkatalysator enthält.

6. Bleichsystem gemäß einem der Ansprüche 1 bis 5, **dadurch gekennzeichnet, dass** das Bleichmittel ausgewählt ist aus Perbenzoesäure, Peroxy-alpha-Napthoesäure, Peroxylaurinsäure, Peroxystearinsäure, Phthalimidoperoxycapronsäure, 6-Phthalimidoperoxyhexansäure (PAP), Nonylimidperoxybernsteinsäure, Nonylimidperoxyadipinsäure, 1,12-Diperoxydodecandisäure, 1,9-Diperoxyazelainsäure, Diperoxoisophthalsäure und 2-Decyldiperoxybutan1,4-disäure.

7. Bleichsystem gemäß einem der Ansprüche 1 bis 6, **dadurch gekennzeichnet, dass** das Polymer erhältlich ist, indem
a) aus Makroolen, ionischen oder potentiell ionischen Polyolen und überschüssigen Polyisocyanaten ein NCO-terminiertes Prepolymer hergestellt wird,
b) dieses Prepolymer mit Verbindungen, die mindestens 2 gegenüber Isocyanat reaktive Aminogruppen aufweisen, in einem Verhältnis NCO-Gruppen/NH-Gruppen von ≤ 1 : 1 umgesetzt und
c) neutralisiert wird.

8. Bleichsystem gemäß Anspruch 7, **dadurch gekennzeichnet, dass** in Schritt c) zur Neutralisierung Ammoniak verwendet wird.

9. Verfahren zur Herstellung eines Bleichsystems gemäß einem der Ansprüche 1 bis 8, **dadurch gekennzeichnet, dass** eine Dispersion enthaltend Polymere, die Urethan-und Harnstoffgruppen aufweisen, auf das Bleichsystem aufgebracht wird.

10. Verfahren gemäß Anspruch 9, **dadurch gekennzeichnet, dass** das Polymer erhältlich ist gemäß Anspruch 7.

11. Reinigerformulierung enthaltend mindestens ein Bleichsystem gemäß einem der Ansprüche 1 bis 8.

12. Reinigerformulierung gemäß Anspruch 11, enthaltend zu
i) 0,1 bis 30 Gew.-% mindestens ein Bleichsystem,
ii) 0,1 bis 99,9 Gew.-% mindestens ein Tensid,
iii) 0 bis 50 Gew.-% mindestens ein Lösungsmittel,
iv) 0 bis 10 Gew.-% mindestens ein Enzym und
v) 0 bis 90 Gew.-% mindestens einen weiteren Zusatzstoff,
wobei das Verhältnis der Komponenten i) bis v) so gewählt ist, dass die Summe 100 Gew.-% ergibt

13. Reinigerformulierung gemäß Anspruch 12, **dadurch gekennzeichnet, dass** das Enzym ausgewählt ist aus der Gruppe der Hydrolasen [EC 3.x.x.x], besonders Esterasen [EC 3.1.x.x] oder Peptidasen [EC 3.4.x.x], vorzugsweise Lipasen [EC 3.1.1.3] oder Subtilisine [EC 3.4.21.62].

14. Reinigerformulierung gemäß einem der Ansprüche 11 bis 13, **dadurch gekennzeichnet, dass** die Formulierung flüssig, gelförmig, pulverförmig, granulär oder tablettenförig ist und/oder das Bleichsystem gegebenenfalls mit anderen Formulierungsbestandteilen in bestimmte Kompartimente der Reinigerformulierung eingearbeitet ist, wobei im Fall von tablettenförmigen Reinigerformulierungen die Kompartimente bestimmte Tablettenschichten und/oder in die Tablette eingelassene, mit der Tablette verklebte oder die Tablette umhüllende Formkörper sein,können.

15. Verwendung eines Bleichsystems gemäß einem der Ansprüche 1 bis 8 oder eine Reinigerformulierung gemäß einem der Ansprüche 11 bis 14 zum Reinigen oder Waschen von Haushaltstextilien oder von Geschirr im Haushaltsbereich oder im gewerblichen Bereich als Fleckensalz, als Desinfektionsmittel und in der Zellstoffbleiche, der Holzstoffbleiche, der Baumwollfaserbleiche oder der Haarbleiche

16. Verwendung gemäß Anspruch 15 in Form eines gelförmigen oder flüssigen Textilwaschmittels oder Reinigers.

## Claims

1. A bleach system comprising at least one component selected from bleach, bleach activator or bleach catalyst, wherein the bleach system is enveloped with at least one polymer layer and the polymer has urethane and urea groups.

2. The bleach system according to claim 1, wherein the bleach system enveloped with at least one polymer layer is particulate.

3. The bleach system according to claim 1 or 2, wherein the mean particle diameter is from 0.01 to 5 mm.

4. The bleach system according to any of claims 1 to 3, wherein the polymer has a layer thickness of from 10 to 2000 µm.

5. The bleach system according to any of claims 1 to 4, wherein the bleach system comprises at least one bleach but no bleach activator and no bleach catalyst.

6. The bleach system according to any of claims 1 to 5, wherein the bleach is selected from perbenzoic acid, peroxy-a-naphthoic acid, peroxylauric acid, peroxystearic acid, phthalimidoperoxycaproic acid, 6-phthalimidoperoxyhexanoic acid (PAP), nonylimidoperoxysuccinic acid, nonylimidoperoxyadipic acid, 1,12-diperoxydodecanedioic acid, 1,9-diperoxyazelaic acid, diperoxoisophthalic acid and 2-decyldiperoxybutane-1,4-dioic acid.

7. The bleach system according to any of claims 1 to 6, wherein the polymer is obtainable by
a) preparing an NCO-terminated prepolymer from macrools, ionic or potentially ionic polyols and excess polyisocyanates,
b) reacting this Prepolymer with compounds which have at least 2 amino groups reactive toward isocyanate in an NCO groups/NH groups ratio of ≤ 1 : 1 and
c) neutralizing it.

8. The bleach system according to claim 7, wherein ammonia is used for neutralization in step c).

9. A process for preparing a bleach system according to any of claims 1 to 8, wherein a dispersion comprising polymers which have urethane and urea groups is applied to the bleach system.

10. The process according to claim 9, wherein the polymer is obtainable according to claim 7.

11. A detergent formulation comprising at least one bleach system according to any of claims 1 to 8.

12. The detergent formulation according to claim 11 comprising
i) from 0.1 to 30% by weight of at least one bleach system,
ii) from 0.1 to 99.9% by weight of at least one surfactant,
iii) from 0 to 50% by weight of at least one solvent,
iv) from 0 to 10% by weight of at least one enzyme and
v) from 0 to 90% by weight of at least one further additive,
the ratio of components i) to v) being selected such that the sum is 100% by weight.

13. The detergent formulation according to claim 12, wherein the enzyme is selected from the group of the hydrolases [EC 3.x.x.x], particularly esterases [EC 3.1.x.x] or peptidases [EC 3.4.x.x], preferably lipases [EC 3.1.1.3] or subtilisins [EC 3.4.21.62].

14. The detergent formulation according to any of claims 11 to 13, wherein the formulation is in liquid, gel, powder, granule or tablet form and/or the bleach system has optionally been incorporated into certain compartments of the detergent formulation with other formulation constituents, the compartments in the case of tableted detergent formulations being certain tablet layers and/or shaped bodies set into the tablet, adhesive-bonded with the tablet or enveloping the tablet.

15. The use of a bleach system according to any of claims 1 to 8 or a detergent formulation according to any of claims 1 to 14 for cleaning or washing household textiles or dishware in the household sector or in the commercial sector, as a stain removal salt, as a disinfectant and in bleaching of mechanical pulp, bleaching of chemical pulp, bleaching of cotton fibers or bleaching of hair.

16. The use according to claim 15 in the form of a gel-form or liquid textile washing composition or detergent.

## Revendications

1. Système de blanchiment, contenant au moins un composant choisi parmi les agents de blanchiment, les activateurs de blanchiment ou les catalyseurs de blanchiment, **caractérisé en ce que** le système de blanchiment est enrobé dans au moins un polymère en forme de couche et le polymère présente des groupes uréthane et urée.

2. Système de blanchiment selon la revendication 1, **caractérisé en ce que** le système de blanchiment enrobé dans au moins un polymère est sous forme de particules.

3. Système de blanchiment selon la revendication 1 ou 2, **caractérisé en ce que** le diamètre moyen des particules est de 0,01 à 5 mm.

4. Système de blanchiment selon l'une quelconque des revendications 1 à 3, **caractérisé en ce que** le polymère présente une épaisseur de couche de 10 à 2000 µm.

5. Système de blanchiment selon l'une quelconque des revendications 1 à 4, **caractérisé en ce que** le système de blanchiment contient au moins un agent de blanchiment, mais pas d'activateur de blanchiment ni de catalyseur de blanchiment.

6. Système de blanchiment selon l'une quelconque des revendications 1 à 5, **caractérisé en ce que** l'agent de blanchiment est choisi parmi l'acide perbenzoïque, l'acide peroxy-alpha-naphtoïque, l'acide peroxylaurique, l'acide peroxystéarique, l'acide phtalimidoperoxycaproïque, l'acide 6-phtalimidoperoxyhexanoïque (PAP), l'acide nonylimidoperoxysuccinique, l'acide nonylimidoperoxyadipique, l'acide 1,12-diperoxydodécanedioïque, l'acide 1,9-diperoxyazélaïque, l'acide diperoxo-isophtalique et l'acide 2-décyldiperoxybutane-1,4-dioïque.

7. Système de blanchiment selon l'une quelconque des revendications 1 à 6, **caractérisé en ce que** le polymère peut être obtenu
a) en préparant, à partir de macro-ols, de polyols ioniques ou potentiellement ioniques et de polyisocyanates en excès, un prépolymère terminé par NCO,
b) en transformant ce prépolymère avec des composés qui présentent au moins deux groupes amino réactifs par rapport à isocyanate, dans un rapport groupes NCO/groupes NH ≤ 1:1 et
c) en neutralisant.

8. Système de blanchiment selon la revendication 7, **caractérisé en ce qu'**on ajoute de l'ammoniaque dans l'étape c) pour la neutralisation.

9. Procédé pour la préparation d'un système de blanchiment selon au moins l'une quelconque des revendications 1 à 8, **caractérisé en ce qu'**on applique une dispersion contenant des polymères, présentant des groupes uréthane et urée, sur le système de blanchiment.

10. Procédé selon la revendication 9, **caractérisé en ce que** le polymère est obtenu selon la revendication 7.

11. Formulation d'agent de nettoyage contenant au moins un système de blanchiment selon l'une quelconque des revendications 1 à 8.

12. Formulation d'agent de nettoyage selon la revendication 11 contenant, à raison de
i) 0,1 à 30% en poids, au moins un système de blanchiment,
ii) 0,1 à 99,9% en poids, au moins un agent tensioactif,
iii) 0% à 50% en poids, au moins un solvant,
iv) 0 à 10% en poids, au moins une enzyme et
v) 0 à 90% en poids, au moins un autre additif,
le rapport des composants i) à v) étant choisi de manière telle que la somme vaut 100% en poids.

13. Formulation d'agent de nettoyage selon la revendication 12, **caractérisée en ce que** l'enzyme est choisie dans le groupe des hydrolases [EC 3.x.x.x], en particulier des estérases [EC 3.1.x.x] ou des peptidases [EC 3.4.x.x], de préférence des lipases [EC 3.1.1.3] ou des subtilisines [EC 3.4.21.62].

14. Formulation d'agent de nettoyage selon l'une quelconque des revendications 11 à 13, **caractérisée en ce que** la formulation est liquide, sous forme de gel, de poudre, de granules ou de comprimés et/ou le système de blanchiment est le cas échéant incorporé avec d'autres constituants de la formulation dans des compartiments déterminés de la formulation de nettoyage, les compartiments, dans le cas de formulations d'agent de nettoyage sous forme de comprimés, étant des couches déterminées du comprimé et/ou des corps façonnés, insérés dans le comprimé, assemblés par collage avec le comprimé ou enrobés par le comprimé.

15. Utilisation d'un système de blanchiment selon l'une quelconque des revendications 1 à 8 ou d'une formulation d'agent de nettoyage selon l'une quelconque des revendications 11 à 14 pour le nettoyage ou le lavage de textiles domestiques ou de vaisselle dans le domaine domestique ou dans le domaine industriel comme sel détachant, comme désinfectant et dans le blanchiment de cellulose, de bois, de fibres de coton ou de cheveux.

16. Utilisation selon la revendication 15 sous forme d'un agent de lavage de textiles ou d'un agent de nettoyage sous forme de gel ou liquide.
